# EUROPEAN PATENT APPLICATION

(11) **EP 2 537 415 A1**
(43) Date of publication of application: **26.12.2012**
(21) Application number: 12173382.8
(22) Date of filing: 25.06.2012
(51) Int. Cl.: A01N 25/10, B01J 47/00

(54) **Biologically efficacious compositions, articles of manufacture and processes for producing and/or using same**

(30) Priority: 24.06.2011 US 201161501086 P; 27.03.2012 US 201261616332 P
(71) Applicant: Vachon, David, Spokane, WA 99223 (US); Knapp, Nathan, Spokane, WA 99207 (US)
(72) Inventor: Vachon, David, Spokane, WA 99223 (US); Knapp, Nathan, Spokane, WA 99207 (US)
(74) Representative: Stuttard, Garry Philip

(57) **Abstract**

Compositions are provided that can include: a polymer matrix; and a plurality of ion-exchange particles distributed within the matrix. Solid polymeric compositions are provided that can include: a plurality of polymer chains from at least one polymer type defining a polymer matrix; and a plurality of polymeric particles distributed within the polymer matrix. Articles of manufacture including a polymeric matrix having a plurality of ion-exchange particles distributed within the matrix are also provided.

Products by process are provided that can include: providing polymer precursors in a substantially unsolidified state; prior to solidifying the precursors, blending the precursors with ion-exchange particles to form a mixture; and solidifying the mixture to form solid polymeric composition product. Solid polymeric composition production methods are also provided that can include: providing a plurality of ion-exchange particles; providing polymer precursors in a substantially unsolidified state; prior to solidifying the precursors, blending the precursors with the ion-exchange particles to form a mixture; and solidifying the mixture to form a solid polymeric composition. Article of manufacture production methods are provided that can include: incorporating a solid polymeric composition into an article of manufacture, the solid polymeric composition including a polymer matrix and a plurality of ion-exchange particles distributed within the polymer matrix.

## Description

### CROSS REFERENCE TO RELATED APPLICATION

This application claims priority to U.S. Provisional Patent Application No. 61/501,086 filed June 24, 2011, entitled "Antimicrobial Materials and Medical Devices" and U.S. Provisional Patent Application No. 61/616,332 filed March 27, 2012, entitled "Antimicrobial Materials and Medical Devices", the entirety of each of which is incorporated by reference herein.

### TECHNICAL FIELD

The disclosure relates generally to biologically efficacious compositions, processes for effecting biological organisms; and/or processes for constructing biologically efficacious articles of manufacture. Embodiments of the disclosure relate to modified polymeric compositions that are antimicrobial and/or the use of these compositions alone or as part of a construction to render all or a portion of the construction antimicrobial.

### BACKGROUND

It can be advantageous to provide materials that are antimicrobial. These materials can be utilized in various situations, but the preparation of these materials as well as their use as part of different constructs provide numerous problems to overcome. The present disclosure provides biologically efficacious compositions, articles of manufacture and processes for producing and/or using same.

### SUMMARY

The disclosure relates generally to compositions that can include ion-exchange particles distributed in a matrix such as a polymer matrix, for example. The particles may be associated with, such as ionically bound to, biologically efficacious materials such as antimicrobial molecular ions and/or metal ions. The bound and/or unbound particles may be utilized as additives and may be readily blended into substrates including the polymeric compositions. Methods for preventing the adhesion of biofilms to material surfaces are also provided. Polymer matrices that may be part of the compositions described herein may include polymers such as silicones, polyurethanes, polyesters, polycarbonates, vinyl polymers (PVC, PVA, PVAc, Polyvinylidene chloride, polyisoprene, SIBS, ABS, SBS, polystyrene, hydrogenated vinyl polymers, e.g. SEBS) & polyalkylenes such as polyethylene, polyamides, epoxies, acrylics, cellulosics, polysaccharides, fluoropolymers, copolymers of various monomers, asphalt, bitumen, and/or blends of these materials for example. The biologically efficacious materials may be incorporated into the composition by using solution chemistry to form a lacquer followed by subsequent evaporation to remove the solvent component of the solution, added to a molten polymer, mixed by shearing, or in the case of silicones, and other materials requiring vulcanization, added to a polymer precursor (prior to vulcanization). The compositions may in addition contain antioxidants, UV stabilizers, fillers, colorants, extrusion aids and/or the like, for example.

As an example, a silicone elastomer can be formulated as an A & B component to be admixed to form a mixture to be readied for vulcanization. The biologically efficacious materials may be added to the component that is devoid of catalyst; as an ion exchange material such as a compound/salt in particle form, for example. The compositions that are formulated to include the additives described herein can be processed to form articles of manufacture that can be formed from coatings (from solvent), extrusion, injection moldings, spun into fiber, or blown into film form. Generally, the compositions described herein may be stable to any variety of processing methodologies including those below 250°C although some of the materials can have stability above 300°C. Compositions of the present disclosure can find applications in construction as many articles of manufacture (paints, flooring, carpets, drywall, caulking and adhesive compounds, counter tops, including laminate materials, filters, appliances), medicine (tubing, molded components, coatings, surgical equipment, biohazard disposal, hospital bedding, gurneys, stretchers textiles that can include clothing to include surgical scrubs, gowns, surgical drapes, bedding, wound dressings), heating, ventilation, & air conditioning (HVAC) system components including filters, heat exchangers, coils, duct work, fans, humidity control components, heat pumps, vents, manifolds and the like (in order to eliminate or control airborne pathogens or bacteria and fungi that can grow in the moist and warm or cool environment of the system), bulk storage containers, public transportation surfaces, office equipment, food conveyers, clean rooms, consumer products (children's toys, high chairs, bathroom cleaning appliances), and hospital, medical & dental office, and military applications such as gear related to the movement of wounded troops from in (battle) theater.

Embodiments of the disclosure provide processes that can provide for the preparation of organic ion and metal ion-modified, stable antimicrobial ion-exchange materials that may be readily added to synthetic plastic, rubber, and natural polymeric matrices.

Example biologically efficacious compositions of the present disclosure can demonstrate effectiveness against a wide-variety of bacterial pathogens and fungi and/or may be incorporated into to various polymeric matrices. For example, quaternary ammonium derivatized ion exchange materials (salts) can be added to 2-part platinum catalyst silicone rubber systems without preventing or inhibiting curing. Quaternary ammonium salts can include the antimicrobial agents benzalkonium chloride and/or cetylpyridinium chloride. These agents have been recognized to inhibit the curing of some thermal-curing and UV-curing polymer materials thus preventing vulcanization. In accordance with example implementations, associating the ammonium ion with a complex anion, can allow the curing to proceed unimpeded.

The disclosure provides a composition that includes a polymer matrix and a plurality of ion-exchange particles distributed within the matrix.

The disclosure further provides a solid polymeric composition that includes a plurality of polymer chains from at least one polymer type defining a polymer matrix and a plurality of polymeric particles distributed within the polymer matrix.

The disclosure also provides an article of manufacture including a polymeric matrix having a plurality of ion-exchange particles distributed within the matrix.

The disclosure provides a product by process that can include providing one or more polymer precursors in a substantially unsolidified state prior to solidifying the precursors, blending the precursors with ion-exchange particles to form a mixture, and solidifying the mixture to form solid polymeric composition product.

The disclosure further provides a solid polymeric composition production method including providing a plurality of ion-exchange particles providing polymer precursors in a substantially unsolidified state prior to solidifying the precursors, blending the precursors with the ion-exchange particles to form a mixture; and solidifying the mixture to form a solid polymeric composition.

The disclosure provides an article of manufacture production method including incorporating a solid polymeric composition into an article of manufacture, the solid polymeric composition including a polymer matrix and a plurality of ion-exchange particles distributed within the polymer matrix.

Compositions of the present disclosure can include heterogeneous polymeric compositions and/or processes using the compositions that can prevent, minimize, and/or eliminate bacterial pathogens, fungi, and their related infection(s) in a host multicellular organism or on surfaces that can host and pass microorganisms onto unsuspecting or susceptible beings.

Compositions for preventing the attachment and colonization of microbes to a polymer surface are also provided that can include an ion exchange material in salt form. The ion exchange material can include cation exchange materials as well as anion exchange materials. These ion exchange materials can also be referred to as resins, such as those in their insoluble, cross linked forms. Cation exchange materials can include weak or strong cation exchange materials for example. Strong cation exchange materials can include polysulfonated salts of polymerized styrene (polystyrene) structure. These polymeric ion exchange materials can be cross linked with divinylbenzene to form insoluble styrene-divinyl\benzene copolymer materials with varying degrees of hydrophilicity (water loving character) depending upon the amount of cross linking agent included. Weak cation exchange materials generally include polycarboxylic acid materials (salts or protonated form) that are generally acrylic structures that may be formed by the polymerization of acrylate materials. These materials can be crosslinked to form insoluble ion exchange materials and the cross linking agents employed may include diacrylates or divinylbenzene to form acrylic-divinylbenzene copolymers for example.

Anion exchange materials can include strongly basic or weakly basic anion exchange materials, for example. Strongly basic anionic exchange materials generally include poly(quaternary ammonium ion) salts and weakly basic anion exchange materials generally include polyamines that are generally secondary amine structures but can include tertiary amines as well. These ion exchange materials can be copolymers of styrene and divinylbenzene, sometimes referred to as styrenedivinylbenzene copolymers. Cation exchange materials as a group can include polymers such as styrene, acrylic, vinyl, sulfonate, carboxylate as well as a variety of counter anions that can include sodium ion, potassium ion, and hydrogen ion, for example. Anion exchange materials can include polymers such as styrene, vinyl, amine, quaternary ammonium as well as counter cations such as chloride ion and hydroxide ion, for example. Cation exchange materials can include cation exchange resins such as polysulfonated salts or polycarboxylated salts of one or more of organic or metal cations.

These materials can be added or blended into a polymer matrix prior to processing into a biologically efficacious composition. The biologically efficacious composition can be incorporated into an article of manufacture and configured to reduce microbial pathogens on the surface and in the space around the article. The counter-ion may include cations that are mono, di, tri, or tetravalent metal ions as well as mono, di, or polyvalent organic cations.

Compositions and/or articles of manufacture described herein can be used for preventing or minimizing inflammation in the tissue surrounding a medical implant. These compositions and/or articles can include a polyamine salt of one or more of organic anions that can be part of a polymer matrix, for example. The compositions and/or articles can also be configured to reduce inflammation around an implant. The polyamine salt can include mono, di, or trivalent organic anions, or polyvalent organic anions.

Compositions and/or articles of manufacture that may provide protection of tissues of a multicellular organism are also provided that can include solid polymer compositions. The solid polymer compositions can be formulated to include an antimicrobial ion containing cation exchange material as well as an anti-inflammatory ion containing anion exchange material, for example.

Processes are also provided for promoting the prevention of microbial (bacterial or fungal) infection or inflammation within a multicellular organism. Minimizing or preventing the colonization of microorganisms within a multicellular organism such as a human being can provide therapeutic benefit to the organism. The processes can include formulating a composition with a therapeutically effective and/or efficacious amount of a cation exchange material such as a polysulfonated material including a bound antimicrobial cation that can be a discreet atomic ion such as a metal cation, an organic molecular cation, or organometallic molecular cation. Embodiments of the processes can be deemed therapeutically efficacious when microbial organisms are prevented from colonizing within the host organism being and/or upon the surface of a medical device; or the microbial organisms are killed once they enter the host organism and as such are prevented from leading to the development of infection and subsequent inflammation; or the microbial organisms are prevented from being transferred from one surface to another or to a host organism, in pathogenic form, such as with a consumer product or by way of a ventilation system for example. The ion exchange material component of the salt may be water-soluble or cross-linked (water insoluble) with the amount (percentage) of crosslinking relating to the amount of water absorption, for example.

Processes are provided for preventing or minimizing bacterial or fungal colonization on the surfaces of articles of manufacture by the incorporation of a cation exchange material that may include incorporating an antimicrobial cation into materials where the colonization of organisms is undesirable.

### DRAWINGS

Embodiments of the disclosure are described below with reference to the following accompanying drawings.

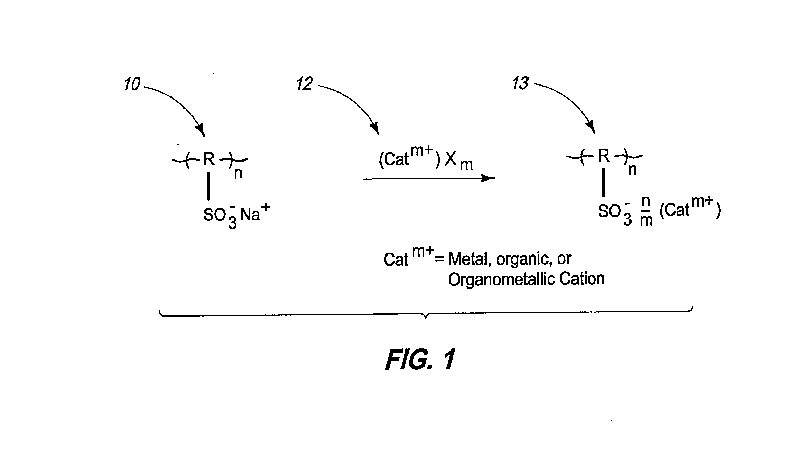

Figure 1 is an example synthetic depiction of a process for preparing compositions according to an embodiment of the present disclosure.

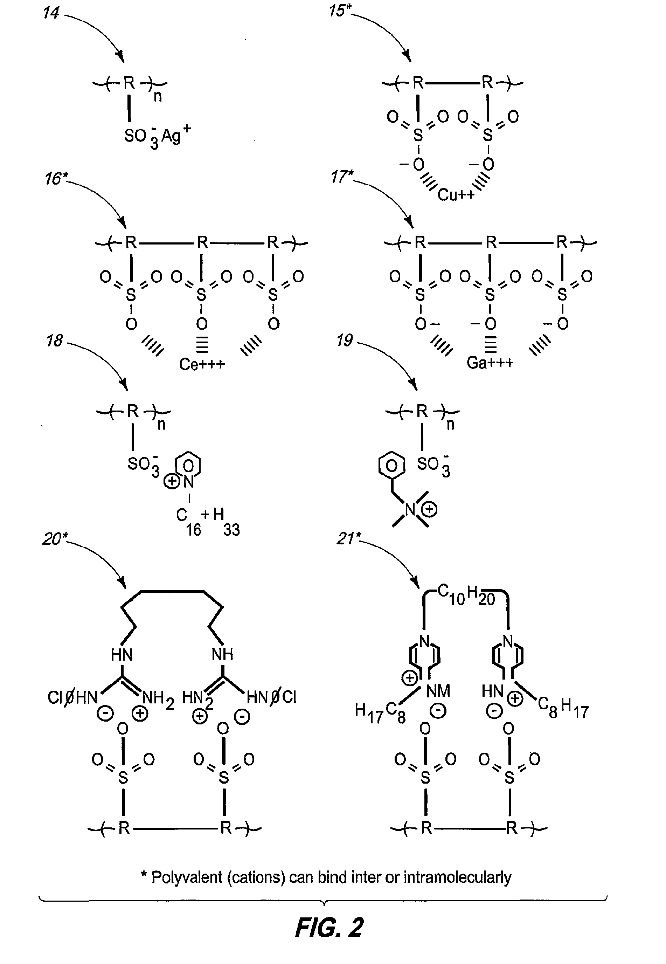

Figure 2 is another example of a process for preparing compositions according to an embodiment of the present disclosure.

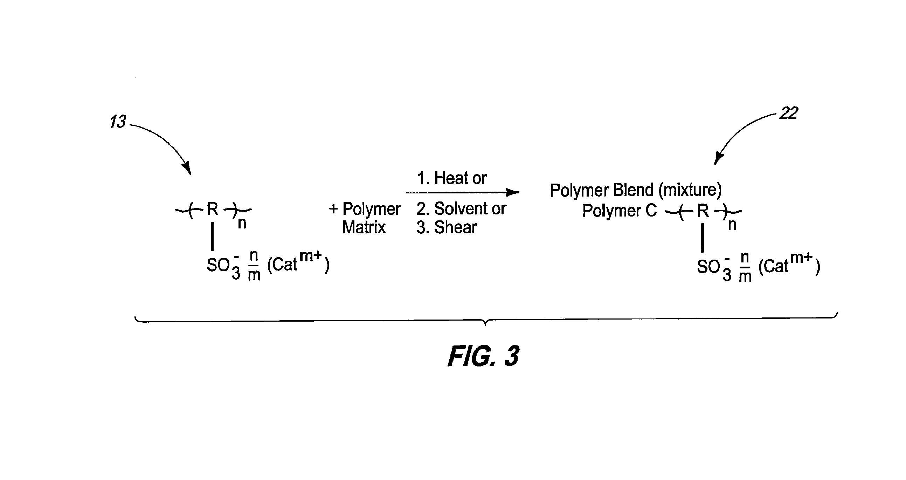

Figure 3 is another example of a process for preparing compositions according to an embodiment of the present disclosure.

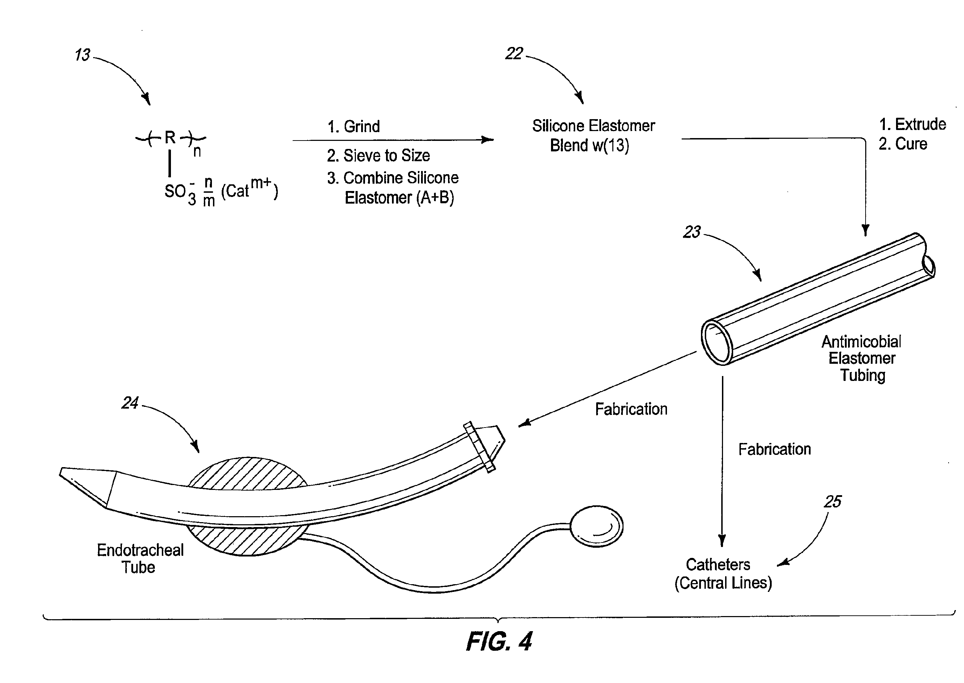

Figure 4 is an example depiction of a process for producing an article of manufacture according to an embodiment of the present disclosure.

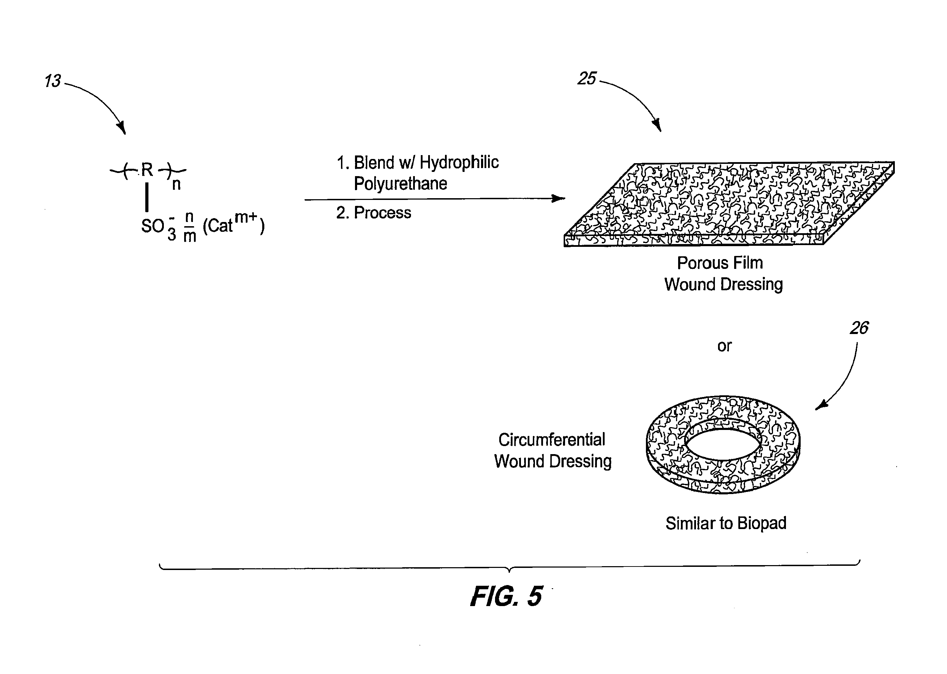

Figure 5 is another example depiction of a process for producing an article of manufacture according to an embodiment of the present disclosure.

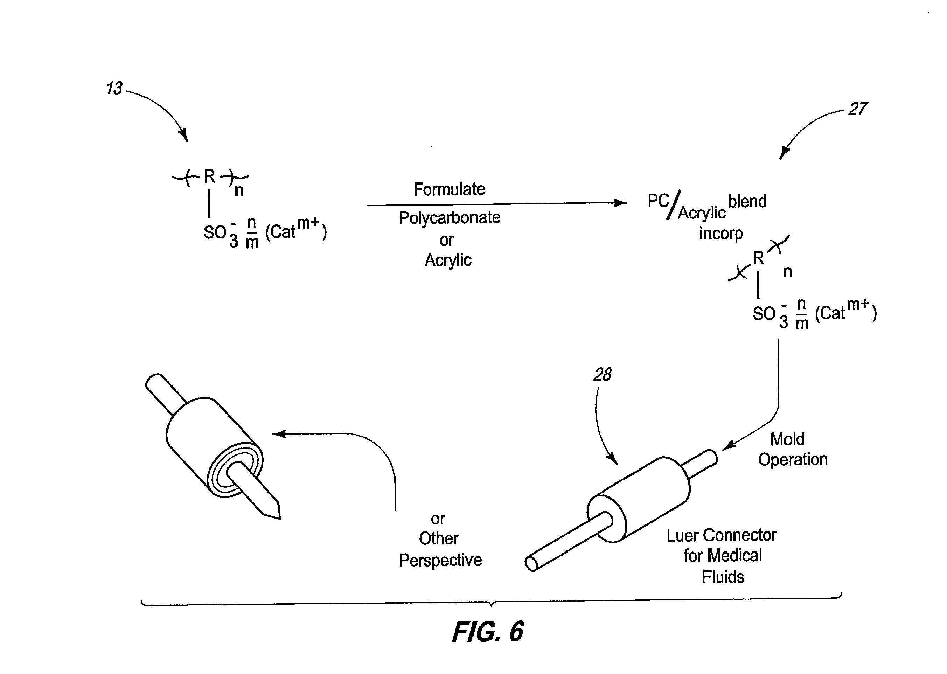

Figure 6 is another example depiction of a process for producing an article of manufacture according to an embodiment of the present disclosure.

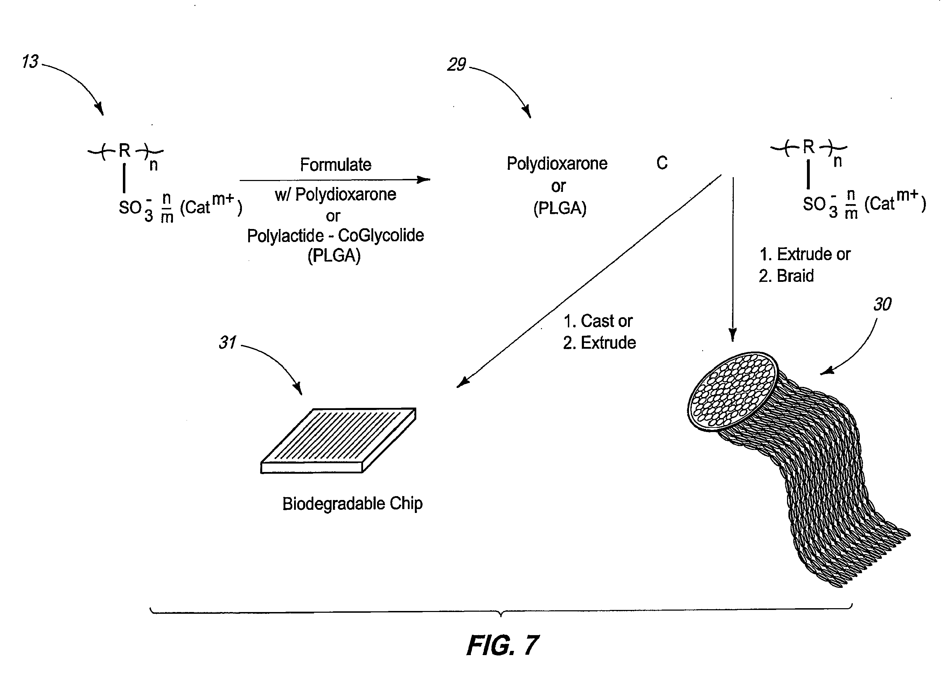

Figure 7 is another example depiction of a process for producing an article of manufacture according to an embodiment of the present disclosure.

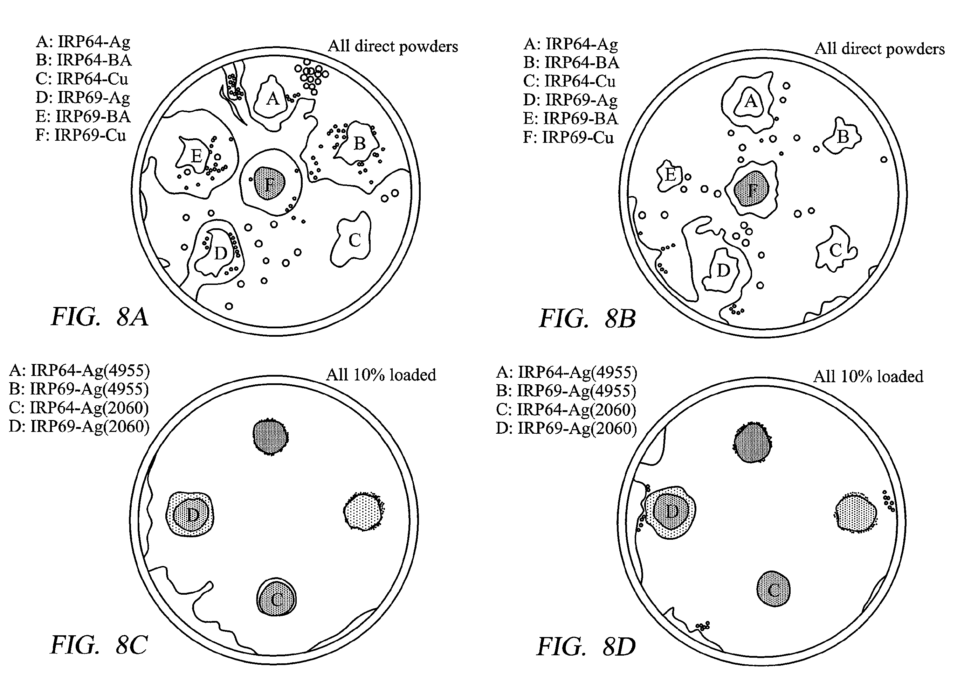

Figure 8A depicts a Kirby-Bauer Assay for (A) Amberlite IRP64-Ag, (B) Amberlite IRP64-Benzalkonium, (C) Amberlite IRP64-Cu(II), (D) Amberlite IRP69- Ag, (E) Amberlite IRP69-Benzalkonium, and (F) Amberlite IRP69-Cu(II) powders vs. *Staphylococcus aureus*.

Figure 8B depicts a Kirby-Bauer Assay for (A) Amberlite IRP64-Ag, (B) Amberlite IRP64-Benzalkonium, (C) Amberlite IRP64-Cu(II), (D) Amberlite IRP69- Ag, (E) Amberlite IRP69-Benzalkonium, and (F) Amberlite IRP69-Cu(II) powders vs. *Pseudomonas aeruginosa.*

Figure 8C depicts a Kirby-Bauer Assay for Amberlite IRP64-Ag and Amberlite IRP69-Ag compounded into (A+B) Nusil MED4955 LSR and (C+D) Momentive UV-curing 2060 LSR materials at 10% w/w vs. *Pseudomonas aeruginosa.*

Figure 8D depicts a Kirby-Bauer Assay for Amberlite IRP64-Ag and Amberlite IRP69-Ag compounded into (A+B) Nusil MED4955 LSR and (C+D) Momentive UV-curing 2060 LSR materials at 10% w/w vs. *Staphylococcus aureus.*

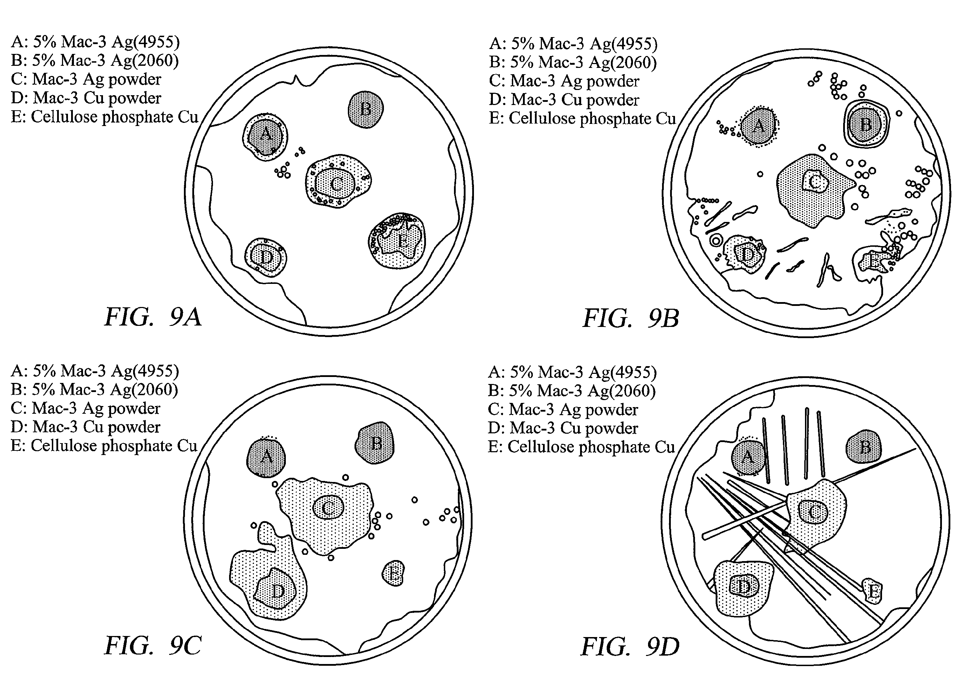

Figure 9A depicts a Kirby-Bauer assay for Mac-3-Ag compounded into (A) Nusil Med 4955 and (B) Momentive UV-curing 2060 LSR materials at 5% w/w, (C) Mac-2-Ag powder, (D) Mac-3-Cu(II) powder, and (E) Cellulose Phosphate-Cu(II) powder vs. *Staphylococcus epidermidis.*

Figure 9B depicts a Kirby-Bauer assay for Mac-3-Ag compounded into (A) Nusil Med 4955 and (B) Momentive UV-curing 2060 LSR materials at 5% w/w, (C) Mac-3-Ag powder, (D) Mac-3-Cu(II) powder, and (E) Cellulose Phosphate-Cu(II) powder vs. *Staphylococcus aureus.*

Figure 9C depicts a Kirby-Bauer assay for Mac-3-Ag compounded into (A) Nusil Med 4955 and (B) Momentive UV-curing 2060 LSR materials at 5% w/w, (C) Mac-3-Ag powder, (D) Mac-3-Cu(II) powder, and (E) Cellulose Phosphate-Cu(II) powder vs. *Pseudomonas aeruginosa.*

Figure 9D depicts a Kirby-Bauer assay for Mac-3-Ag compounded into (a) Nusil Med 4955 and (B) Momentive UV-curing 2060 LSR materials at 5% w/w, (C) Mac-3-Ag powder, (D) Mac-3-Cu(II) powder, and (E) Cellulose Phosphate-Cu(II) powder vs. *Enterococcus faecalis.*

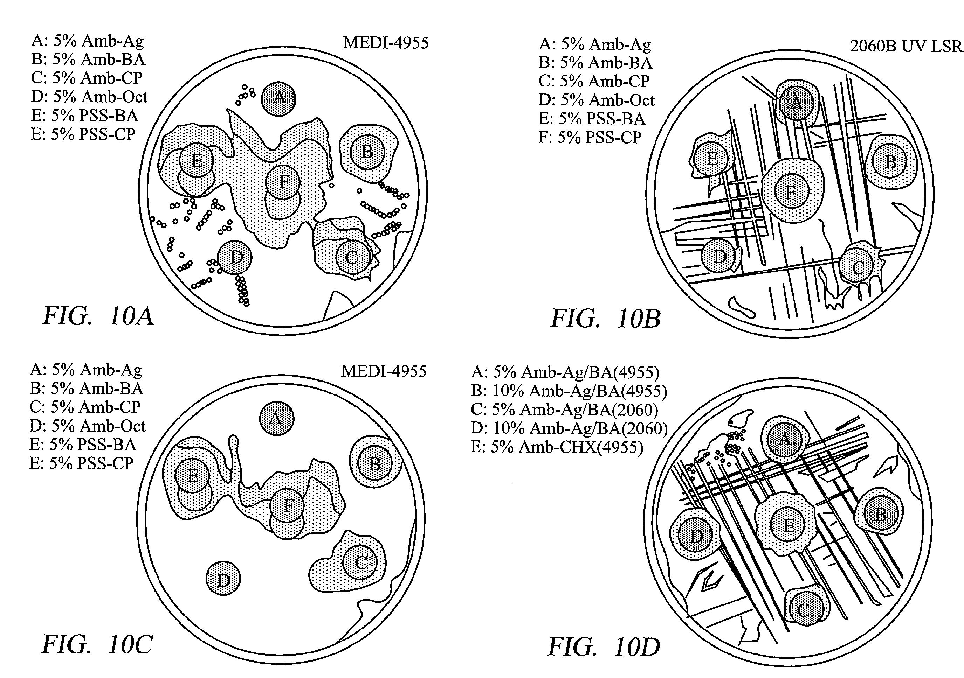

Figure 10A depicts a Kirby-Bauer assay for (A) Amberlite IRP69-Ag, (B) Amberlite IRP69-Benzalkonium, (C) Amberlite IRP69-Cetylpyridinium, (D) Amberlite IRP69-Octenidine, (E) Polystyrene Sulfonate-Benzalkonium, and (F) Polystyrene Sulfonate-Cetylpyridinium formulated into Nusil MED 4955 at 5% w/w against *Staphylococcus aureus.*

Figure 10B depicts a Kirby-Bauer assay for (A) Amberlite IRP69-Ag, (B) Amberlite IRP69-Benzalkonium, (C) Amberlite IRP69-Cetylpyridinium, (D) Amberlite IRP69-Octenidine, (E) Polystyrene Sulfonate-Benzalkonium, and (F) Polystyrene Sulfonate-Cetylpyridinium formulated into Momentive 2060 LSR at 5% w/w against *Staphylococcus aureus.*

Figure 10C depicts a Kirby-Bauer assay for (A) Amberlite IRP69-Ag, (B) Amberlite IRP69-Benzalkonium, (C) Amberlite IRP69-Cetylpyridinium, (D) Amberlite IRP69-Octenidine, (E) Polystyrene Sulfonate-Benzalkonium, and (F) Polystyrene Sulfonate-Cetylpyridinium formulated into Nusil MED 4955 at 5% w/w against *Enterococcus faecalis.*

Figure 10D depicts a Kirby-Bauer assay for (A) Amberlite IRP69-Ag/Benzalkonium combination (5% w/w total in Nusil Med 4955 (2.5% each), (B) Amberlite IRP69-Ag/Benzalkonium (10% w/w total in Nusil Med 4955 (5% each), (C) IRP69-Ag/Benzalkonium combination (5% w/w total in Momentive 2060 UV curing LSR (2.5% each), (D) Amberlite IRP69-Ag/Benzalkonium (10% w/w total in Momentive 2060 UV curing LSR (5% each), Amberlite, and (E) IRP69-chlorhexidine (5% w/w) in Nusil Med 4955 5% w/w against *Staphylococcus aureus.*

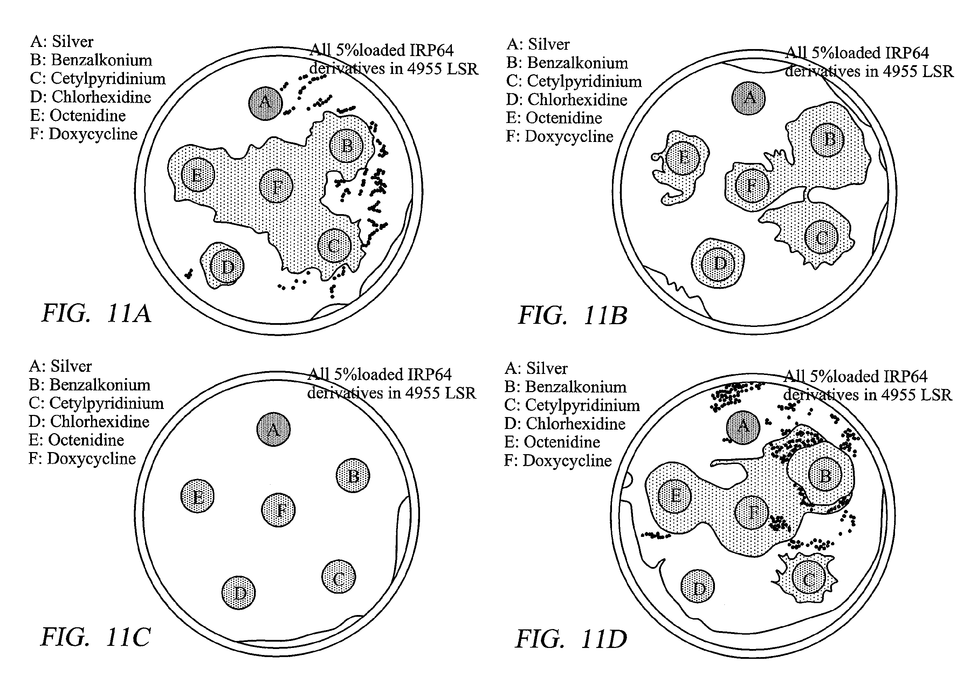

Figure 11A depicts a Kirby-Bauer Assay for (A) Amberlite IRP64-Ag, (B) Amberlite IRP64-Benzalkonium, (C) Amberlite IRP64-Cetylpyridinium, (D) Amberlite IRP64-Chlorhexidine, (E) Amberlite IRP64-Octenidine, and (F) Amberlite IRP64-Doxycycline powders compounded into Med1-4955 vs. *Staphylococcus aureus.*

Figure 11B depicts a Kirby-Bauer Assay for (A) Amberlite IRP64-Ag, (B) Amberlite IRP64-Benzalkonium, (C) Amberlite IRP64-Cetylpyridinium, (D) Amberlite IRP64-Chlorhexidine, (E) Amberlite IRP64-Octenidine, and (F) Amberlite IRP64-Doxycycline powders compounded into Med1-4955 vs. *Staphylococcus epidermidis.*

Figure 11C depicts a Kirby-Bauer Assay for (A) Amberlite IRP64-Ag, (B) Amberlite IRP64-Benzalkonium, (C) Amberlite IRP64-Cetylpyridinium, (D) Amberlite IRP64-Chlorhexidine, (E) Amberlite IRP64-Octenidine, and (F) Amberlite IRP64-Doxycycline powders compounded into Med1-4955 vs. *Pseudomonas aeruginosa*

Figure 11D depicts a Kirby-Bauer Assay for (A) Amberlite IRP64-Ag, (B) Amberlite IRP64-Benzalkonium, (C) Amberlite IRP64-Cetylpyridinium, (D) Amberlite IRP64-Chlorhexidine, (E) Amberlite IRP64-Octenidine, and (F) Amberlite IRP64-Doxycycline powders compounded into Med1-4955 vs. *Enterococcus faecalis.*

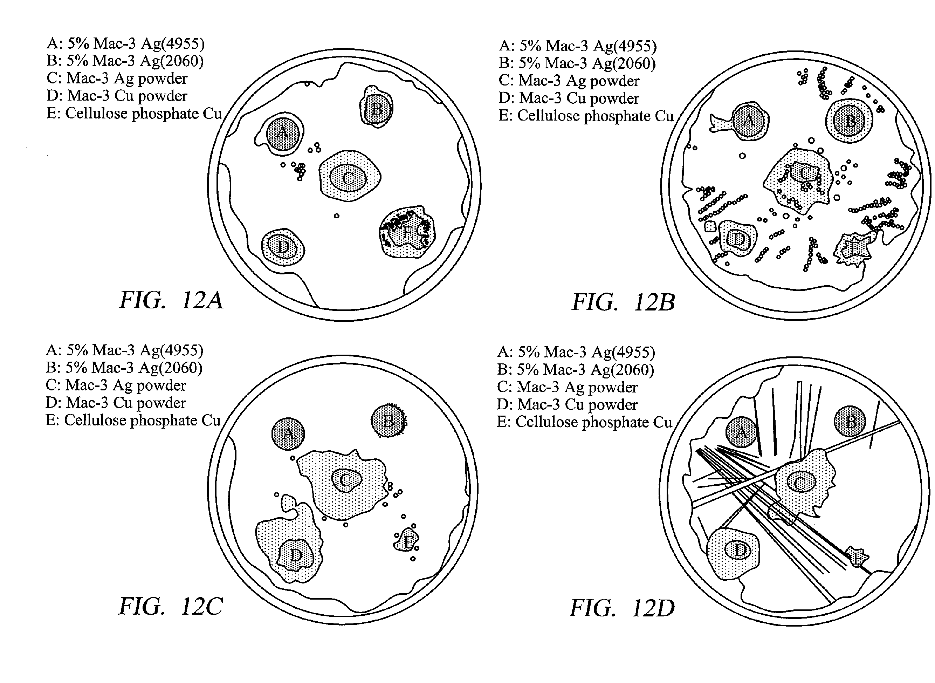

Figure 12A depicts a Kirby-Bauer assay for Mac-3-Ag compounded into (A) Nusil Med 4955 and (B) Momentive UV-curing 2060 LSR materials at 5% w/w, Mac-2-Ag powder, (C) Mac-3-Cu(II) powder, and (D) Cellulose phosphate-Cu(II) powder vs. *Staphylococcus epidermidis.*

Figure 12B depicts a Kirby-Bauer assay for Mac-3-Ag compounded into (A) Nusil Med 4955 and (B) Momentive UV-curing 2060 LSR materials at 5% w/w, (C) Mac-2-Ag powder, (D) Mac-3-Cu(II) powder, and (E) Cellulose Phosphate-Cu(II) powder vs. *Staphylococcus aureus.*

Figure 12C depicts a Kirby-Bauer assay for Mac-3-Ag compounded into (A) Nusil Med 4955 and (B) Momentive UV-curing 2060 LSR materials at 5% w/w, (C) Mac-2-Ag powder, (D) Mac-3-Cu(II) powder, and (E) Cellulose Phosphate-Cu(II) powder vs. *Pseudomonas aeruginosa.*

Figure 12D depicts a Kirby-Bauer assay for Mac-3-Ag compounded into (A) Nusil Med 4955 and (B) Momentive UV-curing LSR materials at 5% w/w, (C) Mac-2-Ag powder, (D) Mac-3-Cu(II) powder, and (E) Cellulose Phosphate-Cu(II) powder vs. *Enterococcus faecalis.*

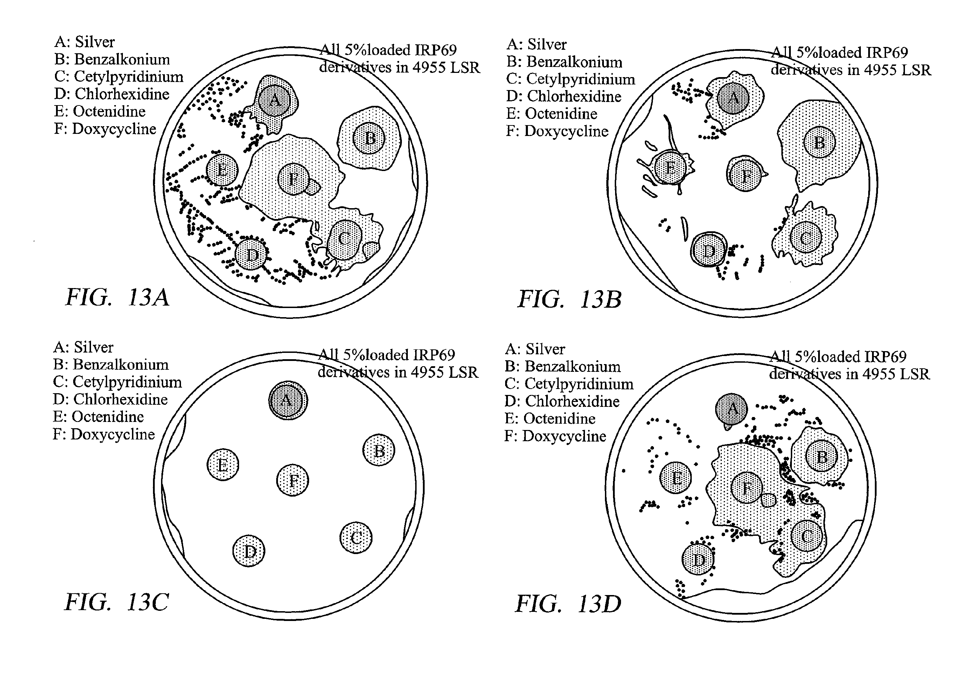

Figure 13A depicts a Kirby-Bauer assay for (A) Amberlite IRP69-Ag, (B) Amberlite IRP69-Benzalkonium, (C) Amberlite IRP69-Cetylpyridinium, (D) Amberlite IRP69-Chlorhexidine, (E) Amberlite IRP69-Octenidine, and (F) Amberlite IRP64-Doxycycline compounded into Nusil MED1-4955 at 5% w/w against *Staphylococcus aureus.*

Figure 13B depicts a Kirby-Bauer assay for (A) Amberlite IRP69-Ag, (B) Amberlite IRP69-Benzalkonium, (C) Amberlite IRP69-Cetylpyridinium, (D) Amberlite IRP69-Chlorhexidine, (E) Amberlite IRP69-Octenidine, and (F) Amberlite IRP64-Doxycycline compounded into Nusil MED1-4955 at 5% w/w against *Staphylococcus epidermidis.*

Figure 13C depicts a Kirby-Bauer assay for (A) Amberlite IRP69-Ag, (B) Amberlite IRP69-Benzalkonium, (C) Amberlite IRP69-Cetylpyridinium, (D) Amberlite IRP69-Chlorhexidine, (E) Amberlite IRP69-Octenidine, and (F) Amberlite IRP64-Doxycycline compounded into Nusil MED1-4955 at 5% w/w against *Pseudomonas aeruginosa.*

Figure 13D depicts a Kirby-Bauer assay for (A) Amberlite IRP69-Ag, (B) Amberlite IRP69-Benzalkonium, (C) Amberlite IRP69-Cetylpyridinium, (D) Amberlite IRP69-Chlorhexidine, (E) Amberlite IRP69-Octenidine, and (F) Amberlite IRP64-Doxycycline compounded into Nusil MED1-4955 at 5% w/w against *Enterococcus faecalis.*

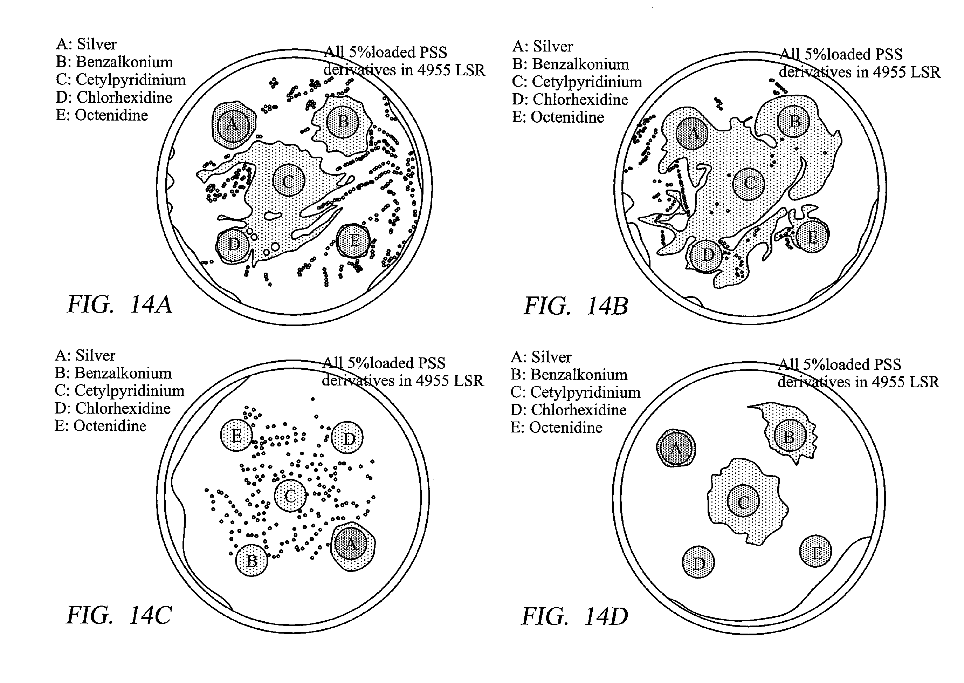

Figure 14A depicts a Kirby-Bauer assay for (A) Polystyrene Sulfonate-Ag, (B) Polystyrene Sulfonate-Benzalkonium, (C) Polystyrene Sulfonate-Cetylpyridinium, (D) Polystyrene Sulfonate-Chlorhexidine, and (E) Polystyrene Sulfonate-Octenidine compounded into Nusil Med1-4955 at 5% w/w against *Staphylococcus aureus.*

Figure 14B depicts a Kirby-Bauer assay for (A) Polystyrene Sulfonate-Ag, (B) Polystyrene Sulfonate-Benzalkonium, (C) Polystyrene Sulfonate-Cetylpyridinium, (D) Polystyrene Sulfonate-Chlorhexidine, and (E) Polystyrene Sulfonate-Octenidine compounded into Nusil Med1-4955 at 5% w/w against *Staphylococcus epidermidis.*

Figure 14C depicts a Kirby-Bauer assay for (A) Polystyrene Sulfonate-Ag, (B) Polystyrene Sulfonate-Benzalkonium, (C) Polystyrene Sulfonate-Cetylpyridinium, (D) Polystyrene Sulfonate-Chlorhexidine, and (E) Polystyrene Sulfonate-Octenidine compounded into Nusil Med1-4955 at 5% w/w against *Pseudomonas aeruginosa.*

Figure 14D depicts a Kirby-Bauer assay for (A) Polystyrene Sulfonate-Ag, (B) Polystyrene Sulfonate-Benzalkonium, (C) Polystyrene Sulfonate-Cetylpyridinium, (D) Polystyrene Sulfonate-Chlorhexidine, and (E) Polystyrene Sulfonate-Octenidine compounded into Nusil Med1-4955 at 5% w/w against *Enterococcus faecalis.*

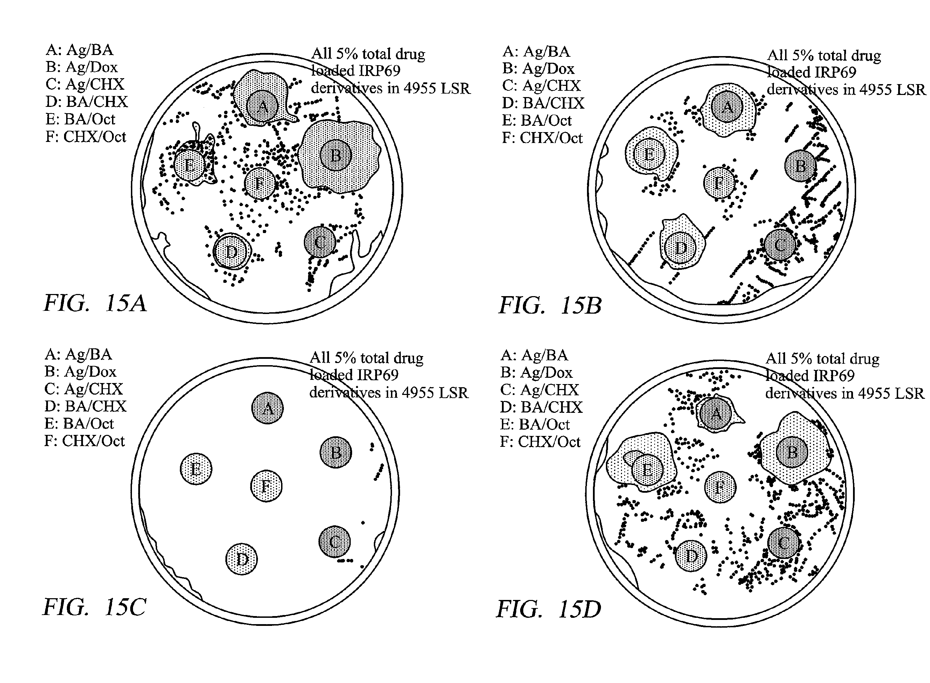

Figure 15A depicts a Kirby-Bauer assay for 50:50 mixed formulations (A) Amberlite IRP69-Ag/Benzalkonium, (B) Amberlite IRP69-Ag/Doxycycline, (C) Amberlite IRP69-Ag/Chlorhexidine, (D) Amberlite IRP69-Benzalkonium/Chlorhexidine, and (E) Amberlite IRP69-Chlorhexidine/Octenidine compounded into Nusil MED1-4955 at 5% w/w (2.5% each) against *Staphylococcus aureus.*

Figure 15B depicts a Kirby-Bauer assay for 50:50 mixed formulations (A) Amberlite IRP69-Ag/Benzalkonium, (B) Amberlite IRP69-Ag/Doxycycline, (C) Amberlite IRP69-Ag/Chlorhexidine, (D) Amberlite IRP69-Benzalkonium/Chlorhexidine, and (E) Amberlite IRP69-Chlorhexidine/Octenidine compounded into Nusil MED1-4955 at 5% w/w (2.5% each) against *Staphylococcus epidermidis.*

Figure 15C depicts a Kirby-Bauer assay for 50:50 mixed formulations (A) Amberlite IRP69-Ag/Benzalkonium, (B) Amberlite IRP69-Ag/Doxycycline, (C) Amberlite IRP69-Ag/Chlorhexidine, (D) Amberlite IRP69-Benzalkonium/Chlorhexidine, and (E) Amberlite IRP69-Chlorhexidine/Octenidine compounded into Nusil MED1-4955 at 5% w/w (2.5% each) against *Pseudomonas aerusinosa.*

Figure 15D. Depicts a Kirby-Bauer assay for 50:50 mixed formulations (A) Amberlite IRP69-Ag/Benzalkonium, (B) Amberlite IRP69-Ag/Doxycycline, (C) Amberlite IRP69-Ag/Chlorhexidine, (D) Amberlite IRP69-Benzalkonium/Chlorhexidine, and (E) Amberlite IRP69-Chlorhexidine/Octenidine compounded into Nusil MED1-4955 at 5% w/w (2.5% each) against *Enterococcus faecalis.*

### DESCRIPTION

This disclosure is submitted in furtherance of the constitutional purposes of the U.S. Patent Laws "to promote the progress of science and useful arts" (Article 1, Section 8).

Compositions are provided than can include a matrix and ion-exchange material within the matrix. The ion-exchange material can be distributed in the matrix. The composition can be a solid polymer composition.

The matrix can be a polymer matrix. The polymer matrix can be one or both of a thermoplastic or thermoset polymer, for example. The polymer matrix can be one or more of a polyalkylene, polysiloxane, polyamide, epoxy, polycarbonate, polyester, polyol, polyarylene, vinyl polymer, acrylic polymer (polyacrylonitrile, polyacrylate), asphalt, bitumen, polysaccharide, cellulosic, and/or polyurethane. The polymer can be at least one polymer from the group consisting of silicone rubber, polyurethane, a polyester, a polycarbonate, a vinyl polymer (PVC, PVA, PVAc, Polyvinylidene chloride, polyisoprene, SIBS, ABS, SBS, polystyrene, hydrogenated vinyl polymers, e.g. SEBS), a polyalkylene such as polyethylene, a polyamide, an epoxy, a phenolic resin, a polyurea, an acrylic, a cellulosic, a fluoropolymer, or a biopolymer such as collagen, hyaluronic acid, gelatin, a hydrogel polymer, and/or an alginate. The polymer matrix can be solid polymer composition. The solid polymer composition can include a plurality of polymer chains from at least one polymer type defining a polymer matrix. The silicone rubber matrix can be one of a silicone rubber adhesive, tacky silicone gel, a liquid silicone rubber, or a high consistency silicone rubber, for example. The matrix when hardened can have a hardness (durometer) in the range of 10 shore A to 65 Shore D.

The ion-exchange material can be a plurality of ion-exchange particles. The ion-exchange material can be polymeric as well such as polymeric particles. The ion-exchange material such as the particles can include one or more of styrene, acrylic, vinyl, sulfonate, quaternary ammonium, amine, and/or carboxylate. The ion-exchange material can be at least one salt of a polysulfonated compound from the group of materials including one or more monomers of an arylenevinyl sulfonate, styrene sulfonate, vinyl sulfonate, or divinyl benzene. The polymeric particles can be dispersed homogenously throughout the matrix, such as the polymer matrix.

The ion-exchange material such as the polysulfonated compound can have a crosslinking unit present between 1 and 15 mole percent. The ion-exchange material such as the polysulfonated compound can have an exchange capacity of between 1.0 and 15.0 mEq/gram. The ion-exchange material such as the polysulfonated compound can be particles having an average particle size between 0.1 and 500 microns. The ion-exchange material can include polysulfonated compounds such as Amberlite IRP69 or IRP88.

Compositions of the present disclosure can include the matrix, the ion-exchange material as well as counter-ion material. The ion-exchange material such as the ion-exchange particles and/or the polymeric particles can be configured to chemically associate with therapeutically efficacious materials. The counter-ion material can be biologically efficacious. For example, the counter-ion material can be antimicrobial and/or anti-inflammatory. As such, the counter-ion material can be therapeutically efficacious in desired applications. The counter-ion material can be chemically associated with at least some of the ion-exchange material such as the particles. Ion-exchange materials can include a metal cation associated with a sulfonate, for example.

The counter-ion material can include one or both of inorganic and organic cations and/or anions. The counter-ion material can be an organic compound containing at least one positively charged nitrogen atom. The counter-ion material can include one or more of a metal cation, quaternary ammonium, organics ions, protonated amines, carboxylate, phosphate, and/or biguanides. The counter-ion material can include one or more of mono, di, and/or trivalent cations. The counter-ion material can include Ag⁺, Cu⁺⁺, Zn⁺⁺, Ga⁺⁺⁺, and/or Fe⁺⁺, for example. The cation can be biologically efficacious such as an antimicrobial cation. In accordance with other example implementations, the metal cation is one or more of Na⁺, Ag⁺, Au⁺, Cu⁺⁺, Ga⁺⁺⁺, Zn⁺⁺, and Ce⁺⁺⁺, Fe⁺⁺, and/or combinations thereof. The solid polymeric composition of claim 11 wherein the polymeric particles are configured to exchange one of a cationic or anionic material.

According to specific implementations, the polymeric composition can include a curable/vulcanizing silicone rubber and the polystyrene sulfonate salt including a metal cation with the cation being associated with 1% to 100% of the sulfonate groups of the polystyrene sulfonate. The salt may include a molecular cation associated with the sulfonate groups incorporated in the range of between 1 and 75 weight%. The polymeric composition can also include a blend of at least two different salts of a polysulfonated compound from the group of materials including one or more monomers of an arylenevinyl sulfonate, styrene sulfonate, vinyl sulfonate, or divinyl benzene with at least one of the polysulfonated compounds including a molecular cation in the range of between 1 and 75 weight % of the product polystyrene sulfonate salt.

In accordance with another implementation, the composition can include a polymeric composition that includes at least one polymer from the group silicone rubber, polyurethane, a polyester, a polycarbonate, a vinyl polymer (PVC, PVA, PVAc, Polyvinylidene chloride, polyisoprene, SIBS, ABS, SBS, polystyrene, hydrogenated vinyl polymers, e.g. SEBS), a polyalkylene such as polyethylene, a polyamide, an epoxy, an acrylic, a cellulosic, a fluoropolymer, or a biopolymer such as collagen, hyaluronic acid, gelatin, a hydrogel polymer, and/or an alginate and at least one salt of a polycarboxylated compound from the group of materials including one or more monomers of an acrylic acid, methacrylic acid, vinylbenzoic acid, arylenevinyl carboxylate, or divinyl benzene and including a metal cation. The metal cation can be oligodynamic and antimicrobial including Ag⁺, Au⁺, Cu⁺⁺, Ga⁺⁺⁺, Zn⁺⁺, and Ce⁺⁺⁺, Fe⁺⁺, and/or combinations thereof. The polymeric composition can include a vulcanizing silicone rubber system and the polycarboxylate salt can be an acrylic acid derivative that includes a metal cation with the metal cation occupying from 1% to 100% of available carboxylates.

In accordance with another implementation, the composition can include a polymeric composition having at least one polymer from the group of silicone rubber, polyurethane, a polyester, a polycarbonate, a vinyl polymer (PVC, PVA, PVAc, Polyvinylidene chloride, polyisoprene, SIBS, ABS, SBS, polystyrene, hydrogenated vinyl polymers, e.g. SEBS), a polyalkylene such as polyethylene, a polyamide, an epoxy, an acrylic, a cellulosic, a fluoropolymer, or a biopolymer such as collagen, hyaluronic acid, gelatin, an alginate and at least one salt of a polycarboxylated compound from the group of materials including one or more monomers of an acrylic acid, methacrylic acid, vinylbenzoic acid, arylenevinyl carboxylate, or divinyl benzene and including a molecular cation. The molecular cation can biologically efficacious such as antimicrobial. The polymeric component can include a vulcanizing silicone rubber system and the polycarboxylated compound can be an acrylic acid salt including an antimicrobial metal cation making up between 1 and 75% w/w.

The composition can include a blend of at least two different salts of a polysulfonated compounds whereby the cation occupies from 5% to 100% of available carboxylates. The composition can include a silicone rubber matrix possessing durometer in the range of 15 shore A to 65 Shore D. The polycarboxylated salt of the polymeric composition can be insoluble in water. The polycarboxylated salt can be a water-insoluble copolymer as a result of a crosslinking copolymer unit. The polycarboxylated salt can be from the group of materials including one or more monomers of acrylic acid, methacrylic acid, vinylbenzoic acid, arylenevinyl carboxylate, or divinyl benzene with the salt possesses an exchange capacity of between 1.0 and 15.0 mEq/gram. The ion-exchange material can be a plurality of particles having sizes between 1 and 500 microns. The polycarboxylated salt can be from the group of materials including one or more monomers of acrylic acid, methacrylic acid, vinylbenzoic acid, arylenevinyl carboxylate, or divinyl benzene, with the salt including a linear water-soluble polycarboxylate. The polycarboxylated salt may also be from the group of materials including one or more monomers of acrylic acid, methacrylic acid, vinylbenzoic acid, arylenevinyl carboxylate, or divinyl benzene with the salt being soluble in water. The polycarboxylated salt of the composition can be one or more of a crosslinked polyacrylic or polymethacrylic acid salt. The polycarboxylated component can be Amberlite IRP64 or MAC-3.

The present disclosure also provides articles of manufacture that can include compositions of the present disclosure. For example, the articles of manufacture can include a matrix such as a polymeric matrix including ion-exchange material such as a plurality of ion-exchange particles distributed within the matrix. The articles can also include counter-ion material such as cationic or anionic material within the polymeric matrix and chemically associated with at least some of the ion-exchange particles.

In accordance with example implementations, the articles of manufacture can include a substrate and the substrate can include the polymeric matrix. As an example, the substrate can have a layer of material thereover, and the layer of material can include the polymeric matrix. The layer of material can be considered a coating and as such the substrate can have at least one surface coated with the polymeric matrix having the ion-exchange material distributed therein. In accordance with some example implementations, the polymeric matrix can define a conduit of the article of manufacture, a vessel of the article of manufacture, and/or a surface of an article of manufacture. The article of manufacture can include medical devices including the polymeric composition with the device being one of a medical tubing, a central venous catheter, pacemaker lead, urological catheter, wound dressing, medical sheeting, endotracheal tube, wound drain, or surgical repair construct or mesh.

The article of manufacture can also include heating, vacuum, or air conditioning components. The heating, vacuum, or air conditioning components can include one or more of duct work, heat exchange coils, heat exchangers, fan components, vents, energy-recovery ventilators, blower components, ballasts, levers, air filters, heat pumps, fluid handling systems and/or the like.

Articles of manufacture can include water filter/purification systems for a pool, hot tub or the like that can include a fluid filter manufactured of an ion exchange material that includes one or more of an antimicrobial metal ion or an organic metal ion in order to control the quantity of microorganisms circulating in the system.

The article of manufacture can include flooring material, a countertop material, a wallboard material, a sealant, an adhesive, a coating, a paint, and a gasket. Articles of manufacture can also include construction wallboard with the construction wall board product including an antimicrobial metal cation including cellulose phosphate additive. The wallboard material can be a drywall product including gypsum.

The present disclosure also provides products according to processes that can include providing one or more polymer precursors in a substantially unsolidified state, and prior to solidifying the precursors, blending the precursors with ion-exchange particles to form a mixture, and solidifying the mixture to form solid polymeric composition product.

The precursors can be one or more of thermoplastic thermoset, elastomer, and/or rigid polymer precursors. The precursors can include one or more of polyalkylene, polysiloxane, polyamide, epoxy, polycarbonate, polyester, polyol, polyarylene, vinyl polymer, acrylic polymer (polyacrylonitrile, polyacrylate), asphalt, bitumen, polysaccharide, cellulosic, and/or polyurethane.

The ion-exchange particles can include polymeric materials such as one or more of styrene, acrylic, vinyl, carboxylate, Sulfonate, quaternary ammonium, and/or amine.

The process can also include associating a cationic or anionic material with the ion-exchange particles. The cationic or anionic material can be therapeutically efficacious. The cationic or anionic material is a mono, di, or trivalent metal. The cationic or anionic material can be substantially inorganic or substantially organic. The cationic or anionic material can include protonated amines, for example.

Composition production methods including solid polymeric composition production methods are also provided. These methods can include: providing a plurality of ion-exchange particles; providing polymer precursors in a substantially unsolidified state; prior to solidifying the precursors, blending the precursors with the ion-exchange particles to form a mixture; and solidifying the mixture to form a solid polymeric composition.

The providing the plurality of ion-exchange particles can include milling a hardened polymer and processing the hardened polymer to provide a plurality of particles ranging in size from about 0.05 microns to about 2500 microns. The milled hardened polymer can include the ion-exchange material associated with or without biologically efficacious counter-ion materials. The hardened polymer can include styrene, acrylic, sulfonate, carboxylate, amine and/or quaternary amine functionalities. The hardened polymer can include a plurality of anionic moieties. These particles can be prepared as salts, for example the particle can be a salt of a hardened polymer ion-exchange material and an organic or inorganic counter-ion.

The providing the polymer precursors in a substantially unsolidified state can include providing nonvulcanized silicone rubber precursors. The blending the precursors with the ion-exchange particles to form a mixture can include providing the particles in an amount between from about 1 to 75% weight percent of the mixture.

The solidifying of the mixture to form a solid polymeric composition can include curing the mixture with the particles uniformly distributed within a portion of the mixture.

The production method can also include providing portions of the polymeric composition free of ion-exchange particles. The method can also include casting the mixture prior to curing. The solidifying of the mixture to form a solid polymeric composition can include evaporating solvent from a polymer lacquer including the particles uniformly distributed within a portion of the mixture.

Article of manufacture production methods are also provided that can include incorporating a solid polymeric composition into an article of manufacture, the solid polymeric composition can include one or more of the compositions described herein such as a polymer matrix and a plurality of ion-exchange particles distributed within the polymer matrix. In accordance with example implementations, the composition can be translucent.

The incorporating can include molding the composition to form a medical device. The incorporating can include molding or extruding the components of the device with the composition to form a catheter.

The incorporating can include preparing the composition as sheets and adhering the sheets to a substrate of the article of manufacture to render at least that portion of the article of manufacture biologically efficacious such as antimicrobial. The incorporating can also include preparing the composition as a coating and adhering the coating to a filtration substrate of the article of manufacture to render at least that portion of the article of manufacture biological efficacious such as antimicrobial. The incorporating can also include preparing the composition as a coating and adhering the coating to a textile substrate of the article of manufacture to render at least that portion of the article of manufacture biologically efficacious such as antimicrobial.

The substrate can be the interior or exterior surface of the article of manufacture. The substrate can be ventilation ducting and the sheet can be adhered to the interior surfaces of the ducting. The substrate can be flooring and the sheet can be adhered to the upper surface of the flooring. The substrate can be carpet and the lower surface of the carpet can include a polymer matrix and a plurality of antimicrobial ion-exchange particles distributed within the polymer matrix.

Compositions such as polymeric compositions and solid polymeric compositions are provided. These compositions can include a polymer matrix as well as ion-exchange materials. The ion-exchange materials can be in the form of ion-exchange particles. The compositions can also include counter ion materials. The Counter ion materials may be biologically efficacious materials.

Compositions and/or articles of manufacture that include biologically efficacious materials are provided. Biologically efficacious materials can include, but are not limited to, antimicrobial agents, such as charged pharmaceutical agents including therapeutic agents or drugs that are effective in the treatment and care of multicellular organisms, chemicals, metal ions or other substances that either improve the biological environment, such as related to aiding healing, reducing inflammation and/or killing or slowing the growth of microbes. Among the antimicrobial agents are antibacterial drugs, including antibiotics, antiviral agents, antifungal agents, organometallic compounds, anti parasitic drugs, and oligodynamic metals to include silver (Ag), for example.

An antimicrobial agent can include a substance that kills or inhibits the growth of microorganisms such as bacteria, fungi, or protozoans. Antimicrobial drugs can kill microbes (microbiocidal) and/or prevent the growth of microbes (microbiostatic). Disinfectants can include antimicrobial substances used on non-living objects or outside the body.

A bactericide or bacteriocide, abbreviated Bcidal, can include a substance that kills bacteria and, in accordance with some implementations, no other living cells are killed. Examples of bactericides include disinfectants, antiseptics or antibiotics.

A bacteriostatic agent or bacteriostat, abbreviated Bstatic, can be an agent that stops (inhibits) bacteria from reproducing, while not necessarily harming them otherwise.

In accordance with example implementations, the biologically efficacious materials can be incorporated into polymer matrixes at temperatures below a point where no damage to the additive occurs. The compositions and/or articles of manufacture described herein can be used to combat microorganism growth and proliferation and these compositions may be water soluble as well as water insoluble depending upon the application.

Furthermore, in the case of water insoluble compositions, insolubility can be descriptive of a component of the composition such as the polysulfonated material component. For example, sodium polystyrene sulfonate and poly(vinyl carboxylic acid), otherwise known as polyacrylic acid, sodium salt are water soluble materials whereas the commercial products Amberlite IRP69, (Rohm and Haas Company, a subsidiary of Dow Chemical Company, Philadelphia, PA 19106-2399), a sulfonated styrene co-divinyl benzene (crosslinked) ion exchange resin and Amberlite IRP64, (Rohm and Haas Company, a subsidiary of Dow Chemical Company, Philadelphia, PA 19106-2399) a polymethacrylic acid, co-divinylbenzene (crosslinked) ion exchange material are both completely insoluble in water by virtue of the divinylbenzene crosslinking of the polymers. The crosslinking monomer, generally divinylbenzene, may be present in the final matrix from 0.1 to 55 mole %. However, acrylic-based ion exchange materials may also be cross linked with a diacrylate molecule. Commercial ion exchange materials can possess on the order of 40 to 55% of divinylbenzene isomers. Generally, the % of crosslinking agent is presented as mol% however it may also be presented as wt% and the amount of swelling (water absorption) and the ion exchange capacity are proportional to the amount of cross linking. For greater the amounts of cross linking, ion exchange capacity and water absorption may decrease for a consistent chemistry such as divinylbenzene cross linked polystyrene sulfonate. IRP69 and IRP64 are reported to possess particle sizes that range from 10-500 mesh (25-1700 micron).

Polymer matrices can include polymers, resins, bitumen, and/or silicones.

Polymers can be described as a class of macromolecules for example. In accordance with some implementations, polymers can include monomeric units of the same structure bonded together or can be include different monomeric structures bonded together, Copolymers can be polymers that can be synthesized with more than one kind of monomeric unit (or monomer). These polymers may be known as homopolymers or copolymers. When a polymer is produced by living organisms, such materials may be referred to as a biopolymer. There are three main classes of biopolymers characterized by their differing monomeric units and/or the structure of the biopolymer formed. Polynucleotides long polymers which can include nucleotide monomers, Polypeptides that can include units of amino acids, and Polysaccharides which may include carbohydrate structures. Cellulose is a very common biopolymer having about 33 percent of all plant matter.

Acrylic can be types of polymeric compounds that contains the acryloyl group derived from acrylic acid, acrylonitrile, acrylamide, or in some instances their derivatives, for example.

Epoxies are generally two-part systems that can include an amine (polyamine) and a polyepoxide. These mateials harden as a consequence of curing which involves the amine component reacting with the epoxide to form a cross linked matrix.

Polyesters are a category of polymers which contain the ester functional group in their main chain.

Polyamides are a category of polymers which contain the amide functional group in their main chain.

Vinyl polymers are polymers origination from vinyl groups. These are alkene derivatives: Primary alkenes contain vinyl groups. On a carbon skeleton, carbon atoms associated with the vinyl group are often called vinylic. Allyls, acrylates and styrenics contain vinyl groups.

The word "resin" has been applied to most components of a liquid that will set into a hard lacquer or enamel-like finish. An example is nail polish, a modern product which contains "resins" that are organic compounds, but not classical plant resins. Certain "casting resins" and synthetic resins (such as epoxy resin) have also been given the name "resin" because they solidify in the same way as some plant resins, but synthetic resins are liquid monomers of thermosetting plastics, and do not derive from plants.

Polyalkylenes are polymers formed by the polymerization of alkenes. The two most common polyalkylenes are the thermoplastic materials polyethylene and polypropylene.

Asphalt, also known as bitumen, is the sticky, black and highly viscous liquid or semi-solid present in most crude petroleums and in some natural deposits; it is a substance classed as a pitch.

A polyurethane (PUR and PU) can be a polymer including carbamate (urethane) linkages.

Silicone rubbers (silicones) can be polymers that generally possess elastomeric properties. Silicones can include silicon together with carbon, hydrogen, and oxygen. Silicone rubbers can be one- or two-part vulcanizing polymer systems that generally cure with the assistance of heat, light, or moisture, and may contain fillers to improve properties or reduce cost. Room temperature vulcanizing formulations or RTVs are silicones that generally cure by the addition of water (moisture) to effect the release of acetic acid, so called acetoxy-curing. However, there are other RTV systems that do not require moisture and rely on a catalyst that is active at room temperature. One part silicone rubber systems can generally cure (vulcanize) at room temperature (RTVs) by acetoxy cure and two part systems generally require heat or light to facilitate action by a platinum or peroxide catalyst. Liquid silicone rubbers or LSRs are two part systems that are more fluid than their high consistency rubber (HCR) counterparts thus providing various processing options to product manufacturers.

Carboxylates can be salts or esters of a carboxylic acid. In the salt form, the carboxylate anion may be paired with metallic or organic cations, for example.

Sulfonates can be salts or esters of a sulfonic acid. The sulfonate functional group can be represented as *R*-SO₂O⁻. It may be paired with a metallic or organic cation, for example.

Quaternary ammonium cations, also known as quats, are positively charged polyatomic ions of the structure NR4+, R being an alkyl group or an aryl group. Unlike the ammonium ion (NH4+) and the primary, secondary, or tertiary ammonium cations, the quaternary ammonium cations are permanently charged, independent of the pH of their solution. Quaternary ammonium salts or quaternary ammonium compounds are salts of quaternary ammonium cations with a counter anion to satisfy the rule of neutrality (salt). Pyridinium refers to the cationic form of pyridine one form of a quaternary ammonium ion. Cetyl pyridinium ion can have a carbon (alkyl) chain of (16) carbons attached to the pyridine nitrogen.

Amines are organic compounds and functional groups that contain a basic nitrogen atom with a lone pair. Amines are derivatives of ammonia, wherein one or more hydrogen atoms have been replaced by a substituent such as an alkyl or aryl group.

Biguanide can refer to a molecule, or to a class of drugs based upon this molecule. Biguanides can function as disinfectants. For example, the biguanides polyaminopropyl biguanide (PAPB) and chlorhexidine both feature biguanide functional groups.

Curing is generally synonymous with vulcanization or crosslinking. Generally, this can include at least a two-part system that includes a catalyst, that when combined with the prepolymer will vulcanize (crosslink) with the addition of energy such as heat or light. However, some single component room temperature vulcanizing (RTV) silicone formulations cure upon exposure to air (moisture).

The phosphate ion is a polyatomic ion with the empirical formula PO₃⁻. In organic chemistry, a phosphate, or organophosphate, is an ester of phosphoric acid. Organic phosphates are important in biochemistry and biogeochemistry or ecology. Inorganic phosphates are mined to obtain phosphorus for use in agriculture and industry.

Durometer is one of several measures expressing the hardness of a material. Hardness may be defined as a material's resistance to permanent indentation. The durometer scale was defined by Albert F. Shore, who developed a measurement device called a durometer in the 1920s. The term durometer is often used to refer to the measurement, as well as the instrument itself. Durometer may be used as a means to measure the hardness in polymers, elastomers, and rubbers.

Weight percent or wt% is a representation of a weight/weight ratio thus indicating the relationship of one material (additive) to another (polymer) for example.

Ion-exchange materials can include ion-exchange resins and/or polymers. Ion-exchange resins can be polymers that are widely used in different separation, purification, and decontamination processes and these materials may be insoluble in water and other solvents. Examples are used in water softening and water purification.

Ion-exchange resins can be based on divinylbenzene crosslinked polystyrene (generally strong cation exchangers and strongly and weakly basic anion exchangers) or divinylbenzene or diacrylate cross linked acrylics (generally weak cation exchangers), for example. The active groups can be introduced after polymerization, or substituted monomers can be used. For example, the crosslinking is often achieved by adding 0.1-55% of divinylbenzene to styrene at the polymerization process. Non-crosslinked polymers are used only rarely because they are less stable and may dissolve when exposed to water or other solvents. Crosslinking can decrease ion-exchange capacity of the resin and may prolong the time needed to accomplish the ion exchange processes. Particle size can also influence the ion-exchange material characteristics; smaller particles have a larger outer surface, but cause larger head loss when utilized, for example.

An ion-exchange material can be an insoluble matrix (or support polymer) which can take the form of small particles or beads on the order of millimeters in diameter or may be used in smaller form on the order of tens to hundreds of microns in diameter. These can be considered ion-exchange particles, for example. These materials may be white or off-white (yellowish) in color, and generally can be fabricated from an organic polymer substrate. These materials can possess highly developed structures of pores on the surface with sites that can trap and release ions. The trapping of ions can take place with simultaneous releasing of other ions; thus the process is referred to as ion-exchange. There are many different types of ion-exchange materials which are fabricated to selectively prefer one or several different types of ions. Generally, ion-exchange functionalities have different selectivities depending upon the ion to be bound and/or exchanged.

Ion exchange capacity or cation exchange capacity (CEC) is one way to represent the maximum quantity of total cations, of any class, that a substance is capable of holding, at a given pH value, for exchange with the eluent that it is in contact with. CEC can be expressed as milliequivalent of cation per 100 g (meq+/100g) of ion exchange material. Amberlite also makes available a product referred to as their IRP 88 series. Larger particle sizes can facilitate filtration of the material following exposure to solutions having the ions of interest. Following modification, the IRP class of materials (IRP69 and IRP64 or the IRP 88 material) can be milled to desired sizes for each application. Milling to average particle sizes of 1-10 microns with such milled samples demonstrating uniform gaussian distributions (as determined by laser particle analysis) can be achieved. Thus, modifying soluble & insoluble ion exchange materials with any biologically efficacious cation such as (Cat)^{m+}, results in materials with two different types of solubility behavior. The IRP69 modified product can release (Cat)^{m+}(X⁺)ₘ in the presence of salt solutions such as NaCl (Na⁺X⁻) such as saline or physiologic fluids. The exchange reaction between sodium polystyrene sulfonate and an organic cation salt or a multivalent metal cation may produce an insoluble salt such as with doxycycline:HCl or gallium nitrate when prepared in deionized water. However upon exposure to saline solution the complex salt can dissolve thus liberating sodium polystyrene sulfonate and doxycycline:HCl and in the case of gallium polystyrene sulfonate, gallium chloride (GaCl₃) and sodium polystyrene sulfonate may be liberated. The modification with any variety of cations (metallic and organic compounds) may be aided with the addition of heat, pressure, the use of columnar flow reactors, and the use of various solvents in addition to/with water.

Compositions, articles of manufacture, and processes of the present disclosure will be described with reference to Figs. 1-15. Referring to Fig. 1, a general synthetic scheme for preparing an ion-exchange material is provided. Starting with an ion-exchange material such as sodium polysulfonate compound 10 and reacting the compound with metal cations 11 or organic cation 12 to produce therapeutically efficacious ion-exchange material such as cation-modified polysulfonate 13. Example efficacious materials can include polysulfonated salts such as the silver (Ag) salt 14, copper (II) (Cu) salt 15, cerium (III) (Ce) salt 16, Gallium (III) (Ga) salt 17, cetylpyridinium (CP) salt 18, benzalkonium (BA) salt 19, chlorhexidine (CHX) salt 20, and octenidine (OCT) salt 21. Other examples can include but are not limited to zinc (II) (Zn), iron (II) (Fe), and minicycline (mino) for example.

Polysulfonated Material 13 can be represented chemically as -(R-SO₃⁻)-ₙ n/m(Cat^{m+}), with n being greater than 1 and the R group containing carbon. Material 13 can be a material that can be associated with various counter ions depicted as (Cat)^{m+} where the number of positive charges is equivalent to the number of sulfonate groups present in the polymer and as such if the cation is a dication for example, it can be associated with more than a single sulfonate group. In the simplest case, (Cat)^{m+} is a proton (H⁺), for example. The ion exchange synthesis of the example efficacious material such as polysulfonated Material 13 is depicted in Figure 1. The R group can be the backbone of an oligomer, such as a dimer and or trimer, or a polymer for example.

Some additional examples of counter cations are shown in Fig. 2. In accordance with other implementations, the oligomer or polymer can include monomers such as arylenevinyl sulfonate, styrene sulfonate, and/or vinyl sulfonate monomers as well as nonsulfonated monomers. The oligomer or polymer can include repeating units of the same monomer, or more than one monomer to simply form a linear copolymer. In other instances the comonomer may serve to crosslink the polymer so as to increase stability and decrease solubility or hydrophilic character such as by the inclusion of a divinylbenzene monomer for example.

According to an example implementation, the oligomer can be combined with other comonomers into other materials. For example, material 13 can also be a copolymer including the oligomer. The oligomer can be copolymerized with other monomers and/or other oligomers to form a copolymer. In accordance with embodiments of the disclosure, the polymer can include repeating oligomer units, whereas the oligomer units may be identical monomer units or mixed monomer units. For example, material 13 can be a crosslinked (water insoluble) polyarylenevinylsulfonate, polystyrene-sulfonate, polyvinylsulfonate, polyalkylenesulfonate polyantholesulfonate, and/or acrylamidomethyl propane sulfonate polymer. In one example, the cross linking monomer can be divinylbenzene.

International Patent Application Number PCTUS0702780 entitled "Compositions and Methods for Promoting the Healing of Tissue of Multicellular Organisms" published July 31^{st}, 2007 describes the preparation of silver Purolite C-160. Purolite C160 is a macroporous poly(styrenesulfonate) cation-exchanger designed to withstand conditions of considerable thermal, osmotic, and mechanical stress. This polymer represents a copolymer (crosslinked) version of material 18 as described in the language & teaching; the entirety of which is incorporated by reference herein. In accordance with other implementations, the oligomer or polymer can include monomers such as arylenevinyl sulfonate, styrene sulfonate, sulfated saccharides, and/or vinyl sulfonate monomers as well as nonsulfonated monomers. The oligomer can include repeating units of the same monomer, or more than one monomer.

For example, material 18 can also be a polymer inlcuding one or more oligomers. The oligomer can be copolymerized with other monomers and/or other oligomers to form a copolymer. In accordance with embodiments of the disclosure, the polymer can include repeating oligomer units, such as polymers of repeating oligomer units. The oligomer units may be identical monomer units or mixed monomer units. For example, material 18 can be polyarylenevinylsulfonate, polystyrene-sulfonate, polyvinylsulfonate, polyantholesulfonate, and/or acrylamidomethyl propane sulfonate polymer.

In accordance with other embodiments of the disclosure, material 18 can be dispersed into a solid matrix of cross-linked acrylic acid-based polymer such as methacrylic acid or any of its esters including poly(2-hydroxy ethyl methacrylate) (HEMA), for example, as well as polypropylene oxide, polyethylene oxide, polyvinyl alcohol, polyurethane, alginate, silicone, hydrocolloid, and/or the hydrogel.

In accordance with an implementation, the sulfonate group can be associated with a complimentary cation. As an example, the sulfonate group can be associated with an inorganic species such as one or more of a monocationic species Na⁺, Ag⁺, K⁺, Li⁺, Au⁺, a dicationic species Ba⁺⁺, Ca⁺⁺, Cu⁺⁺, Zn⁺⁺, Mn⁺⁺, Mg⁺⁺, Fe⁺⁺,or a tricationic species such as Ga⁺⁺⁺, and/or Ce⁺⁺⁺. The sulfonate can also be associated with NH₄⁺, RNH₃⁺, R R^{"}NH₂⁺, RR^{'}R^{"}NH⁺, or⁺, RR'R"R^{"'}N⁺, for example where R represents an aryl, alkyl or mixed aryl alkyl groups or the sulfonate can be associated with a pyridinium cation. According to another example, the sulfonate group can be associated with one or more of organic species including nitrogen containing organic species such as an amino acid, a tetracycline, doxycycline, arginine, lysine, glutathione, lidocaine, albuterol, and/or alkyl/benzylammonium, pyridinium such as cetyl pyridinium, a guanidinium ion such as with chlorhexidine or polyhexanide, amino or oxazole, triazole, ot thiazole containing compounds such as antifungals such as ketoconazole, or clotrimazole, and (dihydropyridinyl) species such as octenidine for example.

In accordance with an example implementation, material 13 can be sodium polystyrene sulfonate, a neutralized derivative of the corresponding polystyrene sulfonic acid. This polymetallosulfonate may be further exchanged with any variety of metal cations to prepare mono, di, tri, and even tetravalent metal salt derivatives. Similarly, poly(metallo)sulfonate such as sodium polystyrene sulfonate, may be converted to a poly(organo)sulfonate derivative by exchange of sodium for any nitrogen atom containing salt/protonatable nitrogen compound of interest. Example nitrogen atom containing salts/protonatable nitrogen compounds can include, but are not limited to, amines, amidines, amidinium ions, imines, thiazoles, imidazoles, guanidines, and/or pyridines. Additionally, ammonium salt derivatives may be prepared by the exposure of an amino compound to the acid form polysulfonate.

In accordance with an embodiment of the disclosure, derivatives of material 13 can be produced by chemically or biochemically modifying material 13. As examples: the cation of material 13 can be modified (substitute Ag⁺ for sodium (Na⁺)); a tetracycline:H⁺ can be substituted for sodium of material 13 via ion exchange; ammonium ion (NH₄⁺) may be substituted for sodium (Na⁺); the sulfonic acid derivative of material 13 may be used as a proton source during an acid-base reaction by treatment with, for example, an amino acid, such as an arginine; a biogenic amine such as tyramine or dopamine. Similarly, the polyanion or polysulfonic acid of material 13 can be exchanged with a polycation or polyamine, such as a strongly basic ion exchange material, for example, poly-L-lysine.

Material 13 can be associated with numerous elements and compounds. For example, material 13 can be associated divalent metal cations Ba⁺², Ca⁺²; Zn⁺²; Cu⁺²; Mg⁺²; Mn⁺², Fe²⁺; trivalent metal cations Ga³⁺; Ce³⁺; or monovalent metal cations Na⁺; Ag⁺; Li⁺; K⁺ or mixed mono-divalent-trivalent compositions. Material 13 can be associated with mixtures of different metal cations, such as mixtures of Cu⁺⁺ and Ag⁺ or Zn⁺⁺ and Ag⁺ for example or mixtures of metallic (inorganic) and molecular cations such as Ag⁺ and NH₄⁺ or Ag⁺, and quaternary ammonium compounds such as benzalkonium for example.

These mixtures can be very beneficial for killing pathogens. In medical applications for example, bacteria can develop resistance to a single agent such as an antimicrobial agent such as chlorhexidine or benzalkonium. However, when one or more of these agents is combined with silver ion (Ag⁺) for example, the differences in mechanisms of killing the bacteria lower the likelihood of the organism developing resistance. Because the ion exchange materials described herein can be readily added to materials as powders, they can be used to easily prepare blended compositions. Because the particle sizes can be adjusted to address cosmetic concerns or adjust availability of ions in the blended material (i.e. surface area for exposure).

Molecular Cations may be polyatomic in nature. These cations may contain organic structures or may be metal ions. Molecular cations examples include ammonium ion (NH₄⁺) or tetracycline⁺.

Referring again to Fig. 1, material 13 is shown as being associated with at least one therapeutic agent (Cat)^{m+} although it is understood that mixtures of cations may be achieved by blending variants (different cation forms) of -(R-SO₃⁻)-ₙ n/m(Cat^{m+})where (Cat)^{m+} differs. By blending two variations of 100% substituted R(SO₃⁻)ₙ(Cat)ⁿ⁺ in solution (i.e. soluble forms), equilibration can yield a 50:50 cation blend in a given sample of the compound. In the case of water insoluble forms of -(R-SO₃⁻)-ₙ n/m(Cat^{m+})physical blends can be utilized for incorporation into any variety of materials to yield mixture 22. Example agents (Cat)^{m+} associated and/or coupled to material 13 are provided as example materials 14-21 of Figure 2. When provided to inhibit bacterial growth, inflammation, or secondary tissue loss that may be associated with traumatic burns, chronic wounds, or infection, the portion of the polysulfonated salt that is the therapeutic agent, (Cat)^{m+}, paired with an appropriate anion (such as Cl⁻), can be released on its own from material 13 including an insoluble (crosslinked) polysulfonated compound thus forming therapeutic agent (Cat)^{m+}(X⁻)ₘ, where X⁻ is chloride (Cl⁻) via ionic exchange, for example. According to an implementation, material 13 can simultaneously provide both proteinase inhibition as well as antimicrobial therapy when the polysulfonated anion is soluble in aqueous (body fluid) media, for example. Insoluble polysulfonated materials can be created generally by the addition/copolymerization of the vinyl derivative, such as styrene, with a di, or trifunctional cross linking agent such as divinyl benzene. Generally, at the lower levels of cross linking, generally indicated as % crosslinker, the polymer may possess some hydrogel-like character whereas at higher crosslink densities the absorption of water can be minimized. The IRP69 derivatives tend to absorb little water (at a reported 8% cross link density) and the little water that is present can be removed under vacuum prior to using these as additives in for example polymeric materials such as silicones or polyurethanes where the addition of a silver derivative may be used to prevent microorganism growth and colonization. Many of the cross linked ion exchange salts are thermally stable thus allowing for drying under vacuum at 150°C.

Furthermore, the insoluble ion exchange material Amberlite IRP69 has an exchange capacity on the order of 4.6 meq/g which can be similar to the very best synthetic zeolite materials (2.5 - 5.5 meq/g). For example, for a fully ion exchanged version of IRP69 where the sodium counter cation is replaced with silver, the final compound possesses approximately 37% Ag by weight. In contrast, zeolites such as the system marketed as AglON (Sciessent LLC, 60 Audobon Road, Wakefield, MA 01880), tend to possess exchange capacities on the order of 2-3 mEq/gram and are generally loaded on the order of less than 20% by weight of silver. Furthermore, zeolites are largely only capable of binding metal cations such as silver ion thus diminishing their utility as a platform for antimicrobial agent delivery. It is also worth noting that many of these materials bind significant amounts of water and they require rigorous drying prior to use. Cosmetically, the polysulfonated salts incorporated into various materials have excellent clarity and color whereas the zeolite formulations tend to be opaque. Amberlite IRP64 has an exchange capacity of 10.0 mEq/g which is superior to the best synthetic zeolites. At 100% of theoretical exchange of sodium ion for silver ion, the resulting silver salt possesses roughly 53% silver by weight. It is believed to be required that at least a portion of the ion exchange particle, sulfonate or carboxylate, retain metal ions having bactericidal properties at ion-exchangeable sites of the ion exchange material in an amount less than the ion-exchange saturation capacity of the ion exchange material. In one embodiment, the ion exchange material employed in the present invention retains antimicrobial metal ions in an amount up to 90% of the theoretical ion-exchange capacity of the ion exchange material. Such ion-exchanged material with a relatively high degree of ion-exchange may be prepared by performing ion-exchange using a metal ion solution having concentrations that are conventionally used for ion exchange. The functionalized ion exchange materials can be milled to uniform particle sizes using for example a centrifugal/planetary ball milling method. As such, homogeneous polymer blends can include uniform particles of modified ion exchange material yield uniform, cosmetically acceptable materials that have demonstrated excellent antimicrobial character over a broad range of additive weight % loadings.

In accordance with example implementations, efficacious ion-exchange materials can be prepared in the form of particles. A mixture of the materials, in the form of particles, for example, and a polymer matrix can be prepared to form a composition of the present disclosure. The mixture may also be processed to form an article of manufacture of the present disclosure.

Referring to Fig. 4, for example, the preparation of a mixture of material 13 with a matrix such as silicone rubber to produce a composition such as mixture 22 may also be processed into an article of manufacture such as extruded tubing 23 and subsequent manufacture to endotracheal tube 24 or catheter 25, for example.

Referring to Fig. 5, as another example, a mixture of strong cation exchange material 13 with a matrix such as polyurethane to produce a composition such as mixture 26 is provided. Mixture 26 can be processed into an article of manufacture such as a wound dressing 27 or a central catheter barrier dressing 28.

Referring to Fig. 6, for example, a mixture of strong cation exchange material 13 with polycarbonate produces mixture 29. Mixture 29 can be further processed into an article of manufacture such as a luer connector 31 for medical fluid/tubing connections.

Referring to Fig. 7, as another example, a mixture of material 13 with a matrix such as polydioxanone produces mixture 32. Mixture 32 can be processed into an article of manufacture such as biodegradable suture 34. In some instances it can be beneficial to utilize a crosslinked (insoluble) material as opposed to a soluble material or *visa versa.* For example, in the case of the biodegradable suture 34 of Fig. 7, the water-soluble ion exchange material may be utilized so that upon degradation of the matrix there remain no insoluble particulates that can lead to a significant foreign body reaction.

Referring to Fig. 4 in which material 13 is formulated into a silicone rubber blend, depicted as a non-vulcanized mixture 22. When the composition includes the polysulfonated compounds 18 and 19 with ion exchange material being Amberlite IRP69, for example, a crosslinked material (polystyrene sulfonate) at several concentrations of compounds 18 & 19 in the matrix (silicone rubber) up to 10% w/w loading, cured under normal curing conditions. Curing (vulcanization) can be accomplished using standard thermal curing silicone elastomers (LSRs, HCRs, and tacky gels from NuSil Technology LLC, 1050 Cindy Lane, Carpinteria, CA 93013) as well as with proprietary UV curing silicone materials (Momentive Performance Materials, 22 Corporate Woods Blvd., Albany, NY 12211-2374). All added compositions remained biological efficacious (antimicrobial) as determined by Kirby Bauer assays against *Staphylococcus epidermidis, Staphylococcus aureus, Pseudomonas aeruginosa,* and *Enterococcus faecalis.*

With attempts to cure the same silicone rubber materials with benzalkonium chloride or cetylpyridinium (CP) chloride alone, none of the materials cured with ammonium salt concentrations as low as 1% w/w loading concentration. Generally, amines, including protonated amines, inhibit the cure of Pt-catalyzed silicone systems as a consequence of poisoning of the catalyst. As such, the material 13 prevents the catalyst from being poisoned by the added amino compound.

Incorporation of compounds 20 and 21 have been accomplished with promising results as well. Chlorhexidine (CHX), which is susceptible to thermal degradation is stabilized when bound to the ion exchange material (polystyrene sulfonate, PSS). Although octenidine hydrochloride can be cured in thermal curing of silicone rubbers, inhibition has been observed at levels of 2-3% and elution of the drug that has been incorporated into the matrix can result in voids within the material. However, the PSS octenidine adduct can improve the cure and allow for much higher molar equivalent amounts of octenidine to be added without interfering with cure.

Kirby-Bauer Assay (Disk diffusion/Zone of inhibition) is a test method that uses antimicrobial-impregnated wafers to test whether particular bacteria are susceptible to specific antimicrobial agents. In this method, bacteria are grown on agar plates in the presence of samples containing relevant antibiotic agents. If the bacteria are susceptible to a particular antibiotic, an area of clearing surrounds the sample where bacteria are not capable of growing (referred to as a zone of inhibition).

Similarly, the anion exchange material Amberlyst A21, a weak base anion exchange material can be associated with an anionic material such as acetylsalicylic acid (ASA) and strongly basic anion exchange resins such as AMBERLITE FPA40-Cl, a food grade strong base anion exchanger for decolorization of sucrose syrups and biopharmaceutical applications, can be used to bind and release dexamethasone sodium phosphate (DexSP) for example. Both ASA and DexSP possess antiinflammatory characteristics. For example, in an attempt to cure ASA directly incorporated into a two-part curing silicone elastomer by mixing, the material was observed not to cure. There has been a desire in the scientific community to deliver this molecule (locally), however the acetyl group has been shown to be labile thus leading to the degradation of ASA to the corresponding phenol compound. However, under mild conditions room temperature aqueous ethanol, ASA can be bound to Amberlyst A21 to yield an ion exchange material including about 30% of the theoretical exchange capacity of the material (4.6 mEq/gram). Following soxhlet extraction (isopropanol) and drying, the material can be incorporated into Nusil MED 4950 (NuSil Technology LLC, 1050 Cindy Lane, Carpinteria, CA 93013) and the cure of the silicone proceeds uninhibited. ASA is readily released from the resulting composition intact, as determined by UV spectroscopy. This ion exchange derivative (Amberlyst A21-ASA) has also been incorporated into Momentive Performance Materials 2060 UV-curing LSR without inhibition of cure. Attempts to cure this silicone rubber material with ASA added directly were unsuccessful as cure was inhibited. For example, the use of ASA in a UV-curing silicone provide the basis for coatings that can be applied to a wide variety of medical devices including hernia repair (tissue repair) mesh materials such as those made from polyester, thus providing a thin coating cross section that eliminates the high (fiber) surface area of the implant, thus improving the foreign body response of such an implant while providing the ability to deliver a non-steroidal antiinflammatory compound. These UV curing silicone materials have proven to be excellent coatings for these supple, yet strong materials while improving their biological effectiveness.

Material 13 may be associated and/or provided with any variety of counter-ion materials such as amine-containing drugs. Amine-containing species include compounds from several classes of therapeutic agents with the majority of all drugs containing at least one basic nitrogen atom within the molecule. Antibiotics and antimicrobial agents, many of which include ammonium groups can be incorporated into silicones and other materials in the form of material 13. The relevant antibiotics that include nitrogen atoms thus allowing salt formation to allow association with material 13 include but are not limited to: Antibiotic Species that are readily protonated (to cationic form) and can be bound to either carboxylated material or sulfonated material systems include: Semisynthetic penicillin such as Ampicillin and Amoxycillin known to be effective against Gram-positive and Gram-negative bacteria by inhibiting steps in cell wall (peptidoglycan) synthesis and murein assembly; the Monobactams to include Aztreonam and produced by the species Chromobacter violaceum and shown to be effective against Gram-positive and Gram-negative bacteria by inhibiting steps in cell wall (peptidoglycan) synthesis and murein assembly; the Carboxypenems to include Imipenem as produced by the species *Streptomyces cattleya* and shown to be effective against Gram-positive and Gram-negative bacteria by inhibiting steps in cell wall (peptidoglycan) synthesis and murein assembly; the Aminoglycosides including Streptomycin as produced by *Streptomyces griseus* shown to be effective against Gram-positive and Gram-negative bacteria. These compounds inhibit translation (protein synthesis) and Gentamicin as produced by the species Micromonospora species shown to be effective against Gram-positive and Gram-negative bacteria especially *Pseudomonas* via Inhibit translation (protein synthesis); the Glycopeptides including Vancomycin as produced by the species *Streptomyces orientales* and shown to be effective Gram-positive bacteria, especially *Staphylococcus aureus* via Inhibiting steps in murein (peptidoglycan) biosynthesis and assembly; the Lincomycins including Clindamycin as produced by Streptomyces lincolnensis and shown to be effective against both Gram-positive and Gram-negative bacteria especially anaerobic Bacteroides by Inhibiting protein synthesis; the Macrolides such as Erythromycin as produced by the species *Streptomyces erythreus* and shown to be effective against Gram-positive bacteria and some Gram-negative bacteria but not enterics, Effective against *Neisseria, Legionella,* and Mycoplasma by Inhibiting protein synthesis; Polypeptides including Polymyxin as produced by the species *Bacillus polymyxa* and known to be effective against Gram-negative bacteria by inducing damage to the cytoplasmic membranes of bacterial species, Bacitracin as produced by the species *Bacillus subtilis* and shown to be effective against Gram-positive bacteria by Inhibiting steps in murein (peptidoglycan) biosynthesis and assembly, Polyenes including Amphotericin as produced by the species *Streptomyces nodosus* and shown to be effective against Fungi by Inactivating membranes containing sterols; Nystatin as produced by the species *Streptomyces noursei* and shown to be effective against Fungi, including *Candida* by Inactivating membranes containing sterols; Rifamycins including Rifampicin as produced by the species *Streptomyces mediterranei* and known to be effective against Gram-positive and Gram-negative bacteria, including *Mycobacterium tuberculosis* by the Inhibition of transcription (eubacterial RNA polymerase); The Tetracyclines as produced by the species *Tetracycline Streptomyces* and shown to be effective against Gram-positive and Gram-negative bacteria, including Rickettsias by the Inhibition of protein synthesis; and the Semisynthetic tetracycline, Doxycycline known to be effective against Gram-positive and Gram-negative bacteria, including *Rickettsias Ehrlichia,* and *Borrelia* via the Inhibition of protein synthesis.

Material 13 may be associated and/or provided with counter-ion materials such as natural and/or synthetic peptides. Material 13 may also be associated with and/or provided in a preparation with: methotrexate; fluorouracil; adriamycin;; ansamitocin; cytosine arabinoside; arabinosyl adenine; mercaptopolylysine; PAM; L-PAM; phenylalanine mustard); mercaptopurine; mitotane; procarbazine dactinomycin (actinomycin D); mitomycin; plicamycin (mithramycin); aminoglutethimide; estramustine; flutamide; leuprolide; megestrol; tamoxifen; amsacrine (m-AMSA); asparaginase (L-asparaginase) Erwina asparaginase; etoposide (VP-16); interferon .alpha.-2a; interferon .alpha.-2b; teniposide (VM-26); adriamycin; arabinosyl; procarbazine; and dacarbazine. In accordance with additional embodiments of the disclosure, Material 13 may also associated with and/or provided in a preparation with: Nitrogen mustards: (Chlorambucil, Chlormethine, Cyclophosphamide, Ifosfamide, Melphalan). Nitrosoureas: (Carmustine, Fotemustine, Lomustine, Streptozocin). Platinum: (Carboplatin, Cisplatin, Oxaliplatin, BBR3464). Busulfan, Dacarbazine, Mechlorethamine, Procarbazine, Temozolomide, ThioTEPA, Uramustine; Antimetabolites: Folic acid: (Methotrexate, Pemetrexed, Raltitrexed). Purine: (Cladribine, Clofarabine, Fludarabine, Mercaptopurine, Thioguanine). Pyrimidine: (Capecitabine). Cytarabine, Fluorouracil, Gemcitabine; Vincaalkaloids: (Vinblastine, Vincristine, Vindesine, Vinorelbine); Cytotoxic/antitumor antibiotics: Anthracycline family: (Daunorubicin, Doxorubicin, Epirubicin, Idarubicin, Mitoxantrone, Valrubicin). Bleomycin, Mitomycin; Topoisomerase inhibitors: Topotecan, Irinotecan; Monoclonal antibodies: Alemtuzumab, Bevacizumab, Cetuximab, Gemtuzumab, Panitumumab, Rituximab, Infliximab, Tositumomab, Trastuzumab, Etanercept; Photosensitizers: Aminolevulinic acid, Methyl aminolevulinate, Porfimer sodium, Verteporfin; Kinase Inhibitors: Dasatinib, Erlotinib, Gefitinib, Imatinib, Lapatinib, Nilotinib, Sorafenib, Sunitinib, Vandetanib (ZD6474).

Additional compounds that may be provided and/or associated with material 13, include: Altretamine, Anagrelide, Bortezomib, Denileukin diftitox, Estramustine, Pentostatin, Pegaspargase, Alagebrium (3-phenacyl-4,5-dimethylthiazolium, anti-helmintics; antitoxins; antivenins;; theophylline; aminophylline; hemin; hematoporphyrins; muramyldipeptide; muramyltripeptide; lymphokines; macrophage activation factor; N-acetyl-muramyl-L-alanyl-D-isoglutamine; ketoconazole; nystatin; griseofulvin; flucytosine (5-fc); miconazole; amphotericin B; ricin; cyclosporins; sulfazecin; growth hormone, melanocyte stimulating hormone; triamcinolone; fludrocortisone; oxytocin; vassopressin; cyanocobalamin; super oxide dismutase; alkaline phosphatase; amelexanox; glutathione; carnosine; p-aminosalicylic acid; isoniazid; capreomycin; cycloserine; ethambutol; ethionamide; pyrazinamide; rifampin; and streptomycin; acyclovir; amantadine azidothymidine; ribavirin and vidarabine; diltiazem; nifedipine; verapamil; dapsone; chloramphenicol; neomycin; cefaclor; cefadroxil; cephalexin; erythromycin; clindamycin; lincomycin; bacampicillin; carbenicillin; dicloxacillin; cyclacillin; picloxacillin; hetacillin; methicillin; nafcillin; oxacillin; penicillins (G&V); ticarcillin; rifampin; doxycycline; mefenamic acid; oxyphenbutazone; phenylbutazone; piroxicam; sulindac; tolmetin; chloroquine; hydroxychloroquine; metronidazole; quinine; quinidine; meglumine; penicillamine; paregoric; codeine; heroin; methadone; morphine; opium; and papaverine; noscapine; deslanoside; atracurium; gallamine; metocurine; pancuronium; succinylcholine (suxamethonium); tubocurarine; vecuronium; ethchlorvynol; flurazepam; glutethimide; methotrimeprazine; methyprylon; midazolam; temazepam; triazolam; bupivacaine; chloroprocaine; etidocaine; lidocaine; mepivacaine; procaine; marcaine; tetracaine; droperidol; etomidate; fentanyl; ketamine; benzyl trimethyl ammonium, chlorhexidine; amino acids (natural & synthetic); nicotinic acid; nicotinamide, pyridoxine; nucleosides (purines); thiamine; coenzyme A; pentoxifylline; 3-amino-4-hydroxybutyric acid; 6-diazo-5-oxo-L-norleucine; aceclofenac; acediasulfone; alminoprofen; amfenac; amoxicillin; ampicillin; apalcillin; apicycline; aspoxicillin; azaserine; aztreonam; bambermycin(s); biapenem; bromfenac; bucillamine; bumadizon; candicidin(s); carbenicillin; carprofen; carumonam; carzinophillin A; ; cefamandole; cefatrizine; cefbuperazone; cefclidin; cefdinir; cefditoren; cefepime; cefetamet; cefixime; cefmenoxime; cefminox; cefodizime; cefonicid; cefoperazone; ceforanide; cefotaxime; cefotetan; cefotiam; cefozopran; cefpimizole; cefpiramide; cefpirome; cefprozil; cefroxadine; ceftazidime; cefteram; ceftibuten; ceftriaxone; cefuzonam; cephaloglycin; cephalosporin C; cephradine; ciprofloxacin; clinafloxacin; cyclacillin; denopterin; diclofenac; edatrexate; enfenamic acid; enoxacin; epicillin; etodolac; flomoxef; flufenamic acid; grepafloxacin; hetacillin; imipenem; lomefloxacin; lymecycline; meclofenamic acid; melphalan; meropenem; moxalactam; mupirocin; mycophenolic acid; nadifloxacin; niflumic acid; norfloxacin; oxaceprol; panipenem; pazufloxacin; penicillin N; pipemidic acid; podophyllinic acid 2-ethylhydrazide; procodazole; pseudoephedrine; pteropterin; quinacillin; ritipenem; romurtide; S-adenosylmethionine; salazosulfadimidine; sparfloxacin; streptonigrin; succisulfone; sulfachrysoidine; sulfaloxic acid; teicoplanin; temafloxacin; temocillin; tetracycline; tolfenamic acid; (N-((5-(((1;4-Dihydro-2-methyl-4-oxo-6-quinazolinyl)methyl)methylamino)-2-thienyl)carbonyl)-L-glutamic acid); tosufloxacin; trovafloxacin; doxyxycline; mafenide; minicycline; tigemonam; or vancomycin; lucensomycin; natamycin or ; 6-diazo-5-oxo-L-norleucine; denopterin; edatrexate; eflomithine; (N-((5-(((1;4-Dihydro-2-methyl-4-oxo-6-quinazolinyl) methyl) methylamino)-2-thienyl)carbonyl)-L-glutamic acid) - ubenimex.

Further drug categories which may be beneficial to incorporate into material 13 and which may provide benefit to incorporated into matrices such as silicone rubber or other materials include antivirals (such as acyclovir, idoxuridine and tromantadine), antimycotics (such as miconazole, ketoconazole, fluconazole, itaconazole, econazole, terconazole, oxyconazole, grisefulvin, clotrimezole, naftifine, and polyenes such as amphotericin B or nystatin/mycostatin), anti-amoebics (such as metronidazole and tinidazole), antihistamines (such as diphenylhydramine, chlorpromazine, pyrilamine and phenyltoloxamine), alkaloids such as morphine, calcium agonists (such as verapamil and nifedipine), anxiolytics, sedatives and hypnotics (such as benzodiazepines, diazepam, nitrazepan, flurazepam, estazolam, flunitrazepam, triazolam, alprazolam, midazolam, temazepam, lormetazepam, brotizolam, clobazam, clonazepam, lorazepam and oxazepam), anti-migraine agents (such as sumatriptan), anti-motion sickness agents (such as cinnarizine), anti-emetics (such as ondansetron, tropisetron and granisetrone), adrenergics (such as amphetamine), antispasmodics (such as aminopentamide, metixene and dicyclomine), ataractics (such as benactyzine), antihypertensives (such as hexamethonium and pentamethonium), analgesics and alkaloids (such as 2,6-diamino-3-phenyl-azopyridine, morphine, papaverine and ethaverine), antitussives (such as dihydrocodeine, phenylpropenolamine, guaiacol, cloperastine and dextromorphen), bronchodilators (such as dimethylephedrine), antipsychotics (such as imipramine), coronary dilators (such as etafenone), antiarrhythmics (such as procainamide), hypotensives (such as hydralazine and clonidine) and peripheral vasoconstrictors (such as tolazoline).

Further examples of amine containing drug compounds include acetophenazine, amitriptyline, bromopheniramine, carbinoxamine, chlorcyclizine, cyclizine, desipramine, dexbrompheniramine, dexchlorpheniramine, ergotamine, nortriptyline, quinidine, benztropine, flunarizine, fluphenazine, hydroxychloroquine, hydroxyzine, meclizine, mesoridine, methdilazine, methysergide, pheniramine, pyrilamine, tripelennamine, triprolidine, promazine and quinidine.

As another example, the ion-exchange material can be a cation exchange material such as cellulose phosphate. This material can include antimicrobial cations such as copper (II) as an example. Cellulose phosphate is a strong cation exchange material of moderate to low ion exchange capacity. This material can be prepared by exposing sodium cellulose phosphate to an excess of copper(II)sulfate in deionized water, filtering and washing the solid until no residual copper(II)sulfate was detected in the filtrate. The material can be tested against Staphylococcus *epidermidis, Staphylococcus aureus, Pseudomonas aeruginosa,* and *Enterococcus faecalis* using a Kirby-Bauer (zone of inhibition) assay. Cellulose Phosphate-Cu⁺⁺ was shown to be effective against the Gram-positive *Staph* bacteria (*epidermidis &* aureus) **(****Figures 9A - 9D****).** However, the ability to release Cu(II) species will also provide excellent effectiveness against fungi. As such, cellulose phosphate materials may provide articles of manufacture such as drywall construction material for example.

The general principles that apply to strong cation exchange materials also apply to weak cation exchange materials. Weak cation exchangers generally contain carboxyl groups (-CO₂⁻) whereas strong cation exchangers contain sulfonates (-SO₃⁻). In general, carboxylates have lower binding constants than do their strong cation exchanger counterparts (sulfonate). As such, carboxylates will give up (exchange/release) dications such as copper (II) and monocations such as silver (I) more readily than their more electronegative counterparts (sulfonates). Experiments that have evaluated the antimicrobial capability of Dow Chemical Dowex MAC-3 (The *Dow Chemical* Company, Midland, Michigan 48674) weak cation exchange material and Amberlite IRP64 (Rohm and Haas Company, a subsidiary of Dow Chemical Company, Philadelphia, PA 19106-2399) weak cation exchange material in Cu(II) and Ag(I) forms were shown to have significant zones of inhibition in Kirby-Bauer assays and the zones were determined to be similar to those of strong cation exchange materials such as Amberlite IRP69 modified to include the same cations of (Cu(II) and Ag(I)) when tested against *Staphylococcus epidermidis, Staphylococcus aureus, Pseudomonas aeruginosa,* and *Enterococcus faecalis.* There are many brands of weak cation exchange materials but generally the carboxyl moiety is introduced via the copolymerization of acrylic acid or methacrylic acid with divinylbenzene or difunctional acrylates thus leading to a polymer with some degree of cross linking. Formulations that exemplify the fabrication of antimicrobial materials from acrylic/methacrylic acid copolymers include Dowex Mac-3 (The Dow Chemical Company, Midland, Michigan 48674) and Amberlite IRP64 (Rohm and Haas Company, a subsidiary of Dow Chemical Company, Philadelphia, PA 19106-2399).

For example, Dowex Mac-3 and IRP64 were modified to include silver ion (See, for example published US patent application No. US20100247544A1 entitled "Compositions and Methods for Promoting the Healing of Tissue of Multicellular Organisms" and published September 30, 2010, the entirety of which is incorporated by reference herein). This silver modified Mac-3 can be dried under vacuum (135°C) to yield an off white solid that was ground to particles and sieved with a 35 micron cutoff sieve. The particles can be dried again under vacuum and formulated into two silicone materials and these materials (silicones with Ag-Mac-3 and the Ag-Mac-3 alone as particles) were evaluated using a Kirby-Bauer assay. Amberlite IRP64 was treated with 0.1N NaOH solution and the sodium salt (Amberlite IRP-64 (Na⁺)) can be filtered and washed with deionized water until the pH of the filtrate was neutral. The salt can be used to prepare Ag⁺, Cu⁺⁺, benzalkonium⁺, chlorhexidine⁺⁺, and octenidine⁺⁺, as well as mixed ion material salts, such as materials incorporating silver and copper or silver and zinc simultaneously. As salts, the particles were incorporated into LSR silicone rubber materials at 5 and 10% loading w/w. Kirby-Bauer assays can be used to evaluate the effectiveness of the materials (ion exchange material loaded with oligodynamic metal ions and ammonium ions, and blended silicone LSR materials) and the materials can be shown to possess broad antimicrobial capability against Gram-negative and Gram-positive organisms, and fungi including but not limited to: *Staphylococcus, Pseudomonas, Escherichia coli, Klebsiella pneumoniae, Legionella, Mycobacteria, Streptococcus, Acinetobacter, Haemophilus,* and *Enterococcus.* As well as *Aspergillis.* These agents can be tailored to address multidrug resistant organisms and a variety of airborne pathogens including *Mycobacterium tuberculosis and Legionella pneumophila.*

Attempts to cure platinum catalyzed silicone rubber formulations compounded with benzalkonium chloride, cetyl pyridinium chloride and other ammonium salts have met with failure. The two-part rubber remained liquid-like of petrolatum consistency. This is the result of inhibition of the catalyst by the amine. It is believed that amines coordinate with such (Pt) catalysts *rendering them inactive and proton donors can affect the same result.* In addition, when curing thermal silicone systems, temperatures of 150°C are required for periods of generally at least five minutes. Because benzalkonium chloride melts at 35°C, the salt particle becomes molten during the attempt to cure thus causing the compound to ooze from the material. However, upon exchange of chloride for polysulfonated anions such as for strong cation exchange materials including non-crosslinked polystyrene sulfonate and crosslinked polystyrene sulfonate such as Amberlite IRP69 or crosslinked carboxylated weak cation exchangers such as Amberlite IRP64, the silicone rubber materials (Nusil MED 4950 thermal curing system and the UV curing system, i.e. Momentive Performance Materials 2060 UV-curing liquid silicone rubber or LSR), demonstrated full cure under normal curing conditions without any melting of the added material and resulted in the absence of any voids in the material. This unexpected result provides for the preparation of silicone rubber materials that do demonstrate zones of inhibition against bacterial species that are relevant to medical device-related infection. Figures 10A - 10C demonstrate the effectiveness of several 5% Amberlite IRP69-Benzalkonium (Amb-BA), Amberlite IRP69-cetyl pyridinium (Amb-CP), and linear (water-soluble) polystyrene sulfonate salts of benzalkonium (PSS-BA) and cetylpyridinium (PSS-CP) formulated into silicone rubbers (Nusil MED 4955 and Momentive 2060 LSR) against *Staphylococcus aureus* and *Enterococcus faecalis.* This exemplifies being able to compound an ammonium salt into a platinum curing silicone system with a non-crosslinked strong cation exchanger (PSS) thus demonstrating the flexibility of this ion-exchange particle approach. The above-mentioned compounds were also effective against *Staphylococcus epidermidis* but less effective against the Gram-negative *Pseudomonas aeruginosa.*

Not all antimicrobial agents (metal ions, organic ions, or organometallic ions) are effective against all bacterial organisms. Generally, Gram-negative species such as *Pseudomonas aeruginosa* are the most difficult to kill. This is exemplified in **Figures 8A-8D** by plates EG133B (*Pseudomonas aeruginosa* and *Staphylococcus aureus*). *Staphylococcus aureus* is a Gram-positive bacterium and the comparative Kirby Bauer demonstrates greater zones against staph. **Figures 8A-8D** also shows plates EG133A are comparisons of the antimicrobial silver ion-containing powders (IRP-64Ag and IRP-69Ag) incorporated into two different LSR elastomers at 10% loading (thermal-curing MED 4955 and UV-curing MPM). The plates show zones of inhibition associated with all samples. Silicone is especially difficult to demonstrate zones of clearance/inhibition around given its hydrophobic nature. Incorporation of the salts into a more hydrophilic compound such as a polyurethane such as Tecoflex EG80A (Lubrizol, example included) can provide different and potentially better release of the active antimicrobial component. Generally, *Pseudomonas* is most aptly dealt with (killed) by silver ion or by mafenide (4-aminomethyl benzenesulfonamide) both of which have been incorporated into the materials discussed herein. As such, it is evident that given the results for MAC-3 **(****Figures 9A - 9D****)** and IRP64 and IRP69 **(****Figures 8A - 8D****),** the strategy disclosed herein can be effective across strong and weak cation exchange materials and performance may be improved by altering the hydrophilicity of the matrix it is incorporated into or in the case where a matrix such as gypsum or as part of the paper coating surface is used (such as in wall board/dry wall), bacterial species may be minimized in situations where the material becomes damp or wet. Similarly, fungi can be limited by the use of copper species of the aforementioned materials discussed herein as fungi are susceptible to Cu(II) salts. Such additives may also provide benefit in coatings used in heating, ventilation, and air conditioning (HVAC) systems). In some cases it may be advantageous to combine such additives with non-corrosive simple salts such as zinc phosphate or sodium propionate in order to aid in the release of the antimicrobial agent in an on-demand fashion inside the HVAC system in the presence of condensed water for example.

ISO 22196 evaluations of antimicrobial ion-modified resins incorporated into a variety of different materials demonstrated between 3-7 log reductions against *Escherichia coli and Staphyloccocus aureus* at as little as 1 wt% loading levels.

The above-data supports the disclosure of carboxylated (weak) cation exchange materials having the ability to provide antimicrobial agents from within material formulations to which they have been incorporated. Aside from the benefit that binding ammonium salts to strong and weak cation exchange polymers provides, curing of silicone elastomers with such antimicrobial compounds, the use of an insoluble ion exchange substrate, vs. the use of antimicrobial compound alone incorporated into the formulation minimizes the void volume that results once the antimicrobial agent, drug elutes away from the ion exchange particle. This phenomenon also provides benefit for (silicone) device applications where a drug eluting from the device can be beneficial and where the drug contains an amine moiety. Thus, minimizing the "void space" that results from the elution of particulate drug is beneficial as this will slow or impede the uptake of moisture by the substrate material.

In addition, binding of a cationic species to the backbone of the ion exchange material can provide one or both of at least two benefits. These include, but are not limited to: 1. Imparting stability to organic cations that may otherwise be thermally unstable thus providing for the active compounds to be incorporated into materials requiring thermal processing such as silicones (vulcanization) or polyurethanes (extrusion, molding); and 2. Active organic salts that are otherwise low temperature melting so as to become liquids at the temperatures required for processing do not melt when bound to the ion exchange material (backbone). For example, benzalkonium chloride (alkyldimethyl(phenylmethyl) quaternary ammonium chloride has a melting point of 34-37°C and a degradation temperature of around 180°C. The melting point is below the processing temperature required for silicone vulcanization or thermal extrusion of most if not all polyurethanes. However, when the quaternary ammonium ion is bound to either a crosslinked polymethacrylic acid or a crosslinked poly(styrene) sulfonic acid it does not melt and maintains stability to approximately 280°C. This data establishes improved thermal stability and improved phase characteristics (i.e. preventing any phase transition such as melting during processing such as molding, extrusion or the like) while the ion exchange material maintains substantial antimicrobial efficacy.

For example, a two part silicone rubber composition suitable for injection molding or extrusion can be compounded to include at least one example of material 13, such as an IRP69-silver derivative, to achieve a homogeneous distribution of material 13 in the silicone composition and the compound can be further molded into an antimicrobial component, such as a suture sleeve for a pacemaker lead. In another example a high consistency rubber composition including material 13, such as IRP69-silver, can be extruded into stand alone tubing or extruded over an existing tube to create a tubing with an active external boundary.

Thus, all figures that apply to material 13 may be substituted to include a carboxyl group (-CO₂⁻) resulting in a structure -(R-CO₂⁻)-ₙ n/m(Cat^{m+}). These materials may be added to a silicone rubber, a polyurethane, a polyester, a polycarbonate, a vinyl polymer (PVC, PVA, PVAc, Polyvinylidene chloride, polyisoprene, Styrene-IsoButylene-Styrene block polymer (SIBS), Acrylonitrile-Butadiene-Styrene (ABS), StyreneButadiene-Styrene (SBS), polystyrene, hydrogenated vinyl polymers, e.g. Styrene-Ethylene-Butylene-Styrene, SEBS), a polyalkylene such as polyethylene, a polyamide, an epoxy, an acrylic, a cellulosic, a fluoropolymer, or a biopolymer such as collagen, hyaluronic acid, gelatin, an alginate.

Example preparations of the compositions of the present disclosure are provided below. These are examples only and are not to be construed to limit the scope of the disclosure provided herein. The claims of the application are supported by the entirety of the disclosure as well as these example. Toward that end, all ion-exchange materials can be purified prior to, and following association with biologically efficacious counter-ion material. Materials as received from the suppliers were soxhlet extracted with isopropyl alcohol and air and vacuum dried.

Following association with the counter-ions, the ion-exchange materials can be milled to predefined sizes into particles. The ion-exchange particles can first be soxhlet extracted with deioinzed water and in a final step soxhlet extracted with isopropyl alcohol. All ion-exchange material can be subsequently air and vacuum dried to remove traces of water and alcohol prior to milling to a desired particle size.
All matrices such as polymer matrices used in the fabrication of the compositions such as silicone rubber, were prepared according to supplier specifications.

Examples:
*1. MED* 6345 *silicone gel (Nusil Silicone Technology, Carpinteria CA 93013) with IRP69-Ag*
   12 g part A + 12 g part B + 2.67 g IRP69-Ag (10 % w/w) mixed in Speedmixer, poured into mold and air bubbles allowed to escape (∼20 min) and cured at 70 °C for 1 hr. Cure was not inhibited.
*2. MED* 6345 *silicone gel (Nusil Silicone Technology, Carpinteria CA 93013) with IRP69-benzalkonium*
   3.07 g part A + 3.07 g part B + 0.3231 g IRP69-BA (5 % w/w) mixed by hand, poured onto release liner and air bubbles allowed to escape (∼20 min) and cured at 84 °C for 23 min. Cure was complete and not inhibited by the ammonium compound.
*3. MED* 6345 *silicone gel (Nusil Silicone Technology, Carpinteria CA 93013) with IRP69-cetylpyridinium*
   3.02 g part A + 3.02 g part B + 0.3179 g IRP69-BA (5 % w/w) mixed by hand, poured onto release liner and air bubbles allowed to escape (∼20 min) and cured at 84 °C for 23 min. Cure was complete and not inhibited by the ammonium compound.
*4. MED* 6345 *silicone gel (Nusil Silicone Technology, Carpinteria CA 93013) with IRP69-octenidine*
   3.015 g part A + 3.015 g part B + 0.3174 g IRP69-BA (5 % w/w) mixed by hand, poured onto release liner and air bubbles allowed to escape (∼20 min) and cured at 84 °C for 50 min. Cure was complete and not inhibited by the ammonium compound. Cure was complete and not inhibited by the ammonium compound.
*5. MED-4955 liquid silicone rubber (Nusil Silicone Technology, Carpinteria* CA *93013) with IRP69-Ag*
   8 g part A + 8 g part B + 0.4948 g IRP69-Ag (3 % w/w) mixed in Speedmixer, spread on release liner and placed in vacuum oven at room temperature to remove air bubbles. Another release liner placed on top, rolled with a jar to flatten and then cured (∼80 °C, ∼10 min). Cure was complete.
*6. MED-4955 liquid silicone rubber (Nusil Silicone Technology, Carpinteria CA 93013) with IRP69-BA*
   18 g part A + 18 g part B mixed in Speedmixer, 6.0844 g removed and 0.3202 g IRP69-BA (5 % w/w) mixed in by hand. Spread on release liner and placed in vacuum oven at room temperature to remove air bubbles. Another release liner placed on top, rolled with a jar to flatten and then cured (∼80 °C, ∼10 min). Cure was complete and not inhibited by the ammonium compound.
*7. MED-4955 liquid silicone rubber (Nusil Silicone Technology, Carpinteria CA 93013) with IRP69-CP*
   9 g part A + 9 g part B mixed in Speedmixer, 6.095 g removed and 0.3208 g IRP69-CP (5 % w/w) mixed in by hand. Spread on release liner and placed in vacuum oven at room temperature to remove air bubbles.
   Another release liner placed on top, rolled with a jar to flatten and then cured (∼80 °C, ∼10 min). Cure was not inhibited. Cure was not inhibited.
*8. MED-4955 liquid silicone rubber with IRP69-Oct*
   12 g part A + 12 g part B mixed in Speedmixer, 6.041 g removed and 0.318 g IRP69-Oct (5 % w/w) mixed in by hand. Spread on release liner and placed in vacuum oven at room temperature to remove air bubbles. Another release liner placed on top, rolled with a jar to flatten and then cured (∼80 °C, ∼10 min). Cure was not inhibited.
*9. Momentive 2060B UV-curing liquid silicone rubber (Momentive Performance Materials, Albany, NY 12211-2374) with IRP69-Ag*
   41.9 g part B + 1.52 g catalyst mixed in Speedmixer, 7.16 g removed and 0.3768 g IRP69-Ag (5 % w/w) mixed in by hand. Spread on release liner and placed in vacuum oven at room temperature to remove air bubbles. Ran through UV curing system (Fusion UV Systems, Inc.) at 4 ft/min; each side of gel exposed to UV lamp once. Cure was not inhibited.
*10. Momentive 20608 UV-curing liquid silicone rubber (Momentive Performance Materials, Albany, NY 12211-2374) with IRP69-BA*
   41.9 g part B + 1.52 g catalyst mixed in Speedmixer, 7.28 g removed and 0.383 g IRP69-BA (5 % w/w) mixed in by hand. Spread on release liner and placed in vacuum oven at room temperature to remove air bubbles. Ran through UV curing system (Fusion UV Systems, Inc.) at 4 ft/min; each side of gel exposed to UV lamp once. Cure was not inhibited.
*11. Momentive 2060B UV-curing liquid silicone rubber (Momentive Performance Materials, Albany, NY 12211-2374) with IRP69-CP*
   41.9 g part B + 1.52 g catalyst mixed in Speedmixer, 7.11 g removed and 0.374 g IRP69-Ag (5 % w/w) mixed in by hand. Spread on release liner and placed in vacuum oven at room temperature to remove air bubbles. Ran through UV curing system (Fusion UV Systems, Inc.) at 4 ft/min; required 4 passes before gel could be removed and turned over, then one more pass on the reverse side. Cure was not inhibited.
*12. Momentive 2060B UV-curing liquid silicone rubber (Momentive Performance Materials, Albany, NY 12211-2374) with IRP69-Oct*
   15 g part B + 0.544 g catalyst mixed in Speedmixer, 6.3327 g removed and 0.3333 g IRP69-Oct (5 % w/w) mixed in by hand. Spread on release liner and placed in vacuum oven at room temperature to remove air bubbles. Ran through UV curing system (Fusion UV Systems, Inc.) at 4 ft/min; each side of gel exposed to UV lamp once.
*13. Tecophilic TG-500 polyurethane (The Lubrizol Corporation, Wickliffe, Ohio 44092) with IRP69-Ag*
   3.01 g Tecophilic SP-80A-150 dissolved in 38.547 mL CHCl₃ on rollermill, 19.995 g soln removed (1.0 g Tecophilic) and mixed with 0.0528 g IRP69-Ag by hand. Poured on release liner to allow CHCl₃ to evaporate.
*14. Tecoflex EG-80A (The Lubrizol Corporation, Wickliffe, Ohio 44092) polyurethane with IRP69-Ag*
   5.012 g Tecoflex MG-8020 dissolved in 64.209 mL CHCl₃ on rollermill, 20.08 g soln removed (1.0 g Tecoflex) and mixed with 0.0528 g IRP69-Ag by hand. Poured on release liner to allow CHCl₃ to evaporate.
*15. Preparation* of salts of *crosslinked polystyrene sulfonates (General Procedure).*
   Amberlite IRP69 strong cation exchange material was stirred in a minimal amount of deionized water and a large excess (∼10-500molar excess) of the salt (containing the cation of interest, such as benzalkonium chloride) was added and the mixture stirred by the addition of a mechanical stirrer for 60 minutes. The solid was filtered washed with copious amounts of deionized water (until the filtrate does not contain any benzalkonium chloride (for example) as evidence from ultraviolet spectroscopic evaluationof the filtrate. The modified IRP69 was dried under vacuum at 130°C and the material was ground with the aid of an IKA homogenizer and the powder put through a sieve with a 35 micron cutoff. The dried powder was dried under vacuum and used for addition to various polymer formulations.
   In an alternate procedure, Amberlite IRP69-Na⁺ (sodium form) strong cation or Amberlite IRP64 exchange material was stirred in a minimal amount of deionized water and an equal amount of isopropanol is added. A large excess (∼10-500molar excess) of the salt (containing the cation of interest, such as benzalkonium chloride) was added and the mixture was heated to 65°C (optimal temperatures are between 55-70°C) and the mixture is continuously stirred with a mechanical stirrer for up to 72 hours. The solid ion exchange material was subsequently filtered washed with isopropanol and deionized water (until the filtrate does not contain any benzalkonium chloride (for example) as evidence from ultraviolet spectroscopic evaluation of the filtrate. The modified IRP69 or IRP64 was dried under vacuum at 130°C and the resin are easily ground with the aid of a planetary ball mill such as a Retsch PM100 and the appropriate grinding media. The materials can be routinely milled to 1-10 micron particle sizes as measured by light scattering.
*16. MED 6345 silicone gel (Nusil Silicone Technology, Carpinteria CA 93013) with IRP64-Ag*
   12 g part A + 12 g part B + 2.67 g IRP64-Ag (10 % w/w) mixed in Speedmixer, poured into mold and air bubbles allowed to escape (∼20 min) and cured at 70 °C for 1 hr. Cure was not inhibited.
*17. MED* 6345 *silicone gel (Nusil Silicone Technology, Carpinteria CA 93013) with IRP64-benzalkonium*
   3.07 g part A + 3.07 g part B + 0.3231 g IRP64-BA (5 % w/w) mixed by hand, poured onto release liner and air bubbles allowed to escape (∼20 min) and cured at 84 °C for 23 min. Cure was complete and not inhibited by the ammonium compound.
*18. MED* 6345 *silicone gel (Nusil Silicone Technology, Carpinteria CA 93013) with IRP64-cetylpyridinium*
   3.00 g part A + 3.00 g part B + 0.3305 g IRP64-BA (5 % w/w) mixed by hand, poured onto release liner and air bubbles allowed to escape (∼20 min) and cured at 84 °C for 23 min. Cure was complete and not inhibited by the ammonium compound.
*19. MED* 6345 *silicone gel (Nusil Silicone Technology, Carpinteria CA 93013) with IRP64-octenidine*
   3.060 g part A + 3.060 g part B + 0.3255 g IRP64-BA (5 % w/w) mixed by hand, poured onto release liner and air bubbles allowed to escape (∼20 min) and cured at 84 °C for 50 min. Cure was complete and not inhibited by the ammonium compound. Cure was complete and not inhibited by the ammonium compound.
*20. MED-4955 liquid silicone rubber (Nusil Silicone Technology, Carpinteria CA 93013) with IRP64-Ag*
   7.9 g part A + 7.9 g part B + 0.4997 g IRP64-Ag (3 % w/w) mixed in Speedmixer, spread on release liner and placed in vacuum oven at room temperature to remove air bubbles. Another release liner placed on top, rolled with a jar to flatten and then cured (∼80 °C, ∼10 min). Cure was complete.
*21. MED-4955 liquid silicone rubber (Nusil Silicone Technology, Carpinteria CA 93013) with IRP64-BA*
   18.2 g part A + 18.2 g part B mixed in Speedmixer, 6.214 g removed and 0.3202 g IRP64-BA (5 % w/w) mixed in by hand. Spread on release liner and placed in vacuum oven at room temperature to remove air bubbles. Another release liner placed on top, rolled with a jar to flatten and then cured (∼80 °C, ∼10 min). Cure was complete and not inhibited by the ammonium compound.
*22. MED-4955 liquid silicone rubber (Nusil Silicone Technology, Carpinteria CA 93013) with IRP64-CP*
   9.1 g part A + 9.1 g part B mixed in Speedmixer, 6.195 g removed and 0.3208 g IRP64-CP (5 % w/w) mixed in by hand. Spread on release liner and placed in vacuum oven at room temperature to remove air bubbles. Another release liner placed on top, rolled with a jar to flatten and then cured (∼80 °C, ∼10 min). Cure was not inhibited. Cure was not inhibited.
*23. MED-4955 liquid silicone rubber with IRP64-Oct*
   12 g part A + 12 g part B mixed in Speedmixer, 6.041 g removed and 0.318 g IRP64-Oct (5 % w/w) mixed in by hand. Spread on release liner and placed in vacuum oven at room temperature to remove air bubbles. Another release liner placed on top, rolled with a jar to flatten and then cured (∼80 °C, ∼10 min). Cure was not inhibited.
*24. Momentive 2060B UV-curing liquid silicone rubber with IRP64-Ag*
   41.9 g part B + 1.52 g catalyst mixed in Speedmixer, 7.16 g removed and 0.3768 g IRP64-Ag (5 % w/w) mixed in by hand. Spread on release liner and placed in vacuum oven at room temperature to remove air bubbles. Ran through UV curing system (Fusion UV Systems, Inc.) at 4 ft/min; each side of gel exposed to UV lamp once. Cure was not inhibited.
*25. Momentive 2060B UV-curing liquid silicone rubber with IRP64-BA*
   41.9 g part B + 1.52 g catalyst mixed in Speedmixer, 7.28 g removed and 0.383 g IRP64-BA (5 % w/w) mixed in by hand. Spread on release liner and placed in vacuum oven at room temperature to remove air bubbles. Ran through UV curing system (Fusion UV Systems, Inc.) at 4 ft/min; each side of gel exposed to UV lamp once. Cure was not inhibited.
*26. Momentive 2060B UV-curing liquid silicone rubber with IRP64-CP*
   41.9 g part B + 1.52 g catalyst mixed in Speedmixer, 7.11 g removed and 0.374 g IRP64-Ag (5 % w/w) mixed in by hand. Spread on release liner and placed in vacuum oven at room temperature to remove air bubbles. Ran through UV curing system (Fusion UV Systems, Inc.) at 4 ft/min; required 4 passes before gel could be removed and turned over, then one more pass on the reverse side. Cure was not inhibited.
*27. Momentive 2060B UV-curing liquid silicone rubber with IRP64-Oct*
   15 g part B + 0.544 g catalyst mixed in Speedmixer, 6.3327 g removed and 0.3333 g IRP64-Oct (5 % w/w) mixed in by hand. Spread on release liner and placed in vacuum oven at room temperature to remove air bubbles.
   Ran through UV curing system (Fusion UV Systems, Inc.) at 4 ft/min; each side of gel exposed to UV lamp once.
*28. Tecophilic TG-500 polyurethane with IRP64-Ag*
   3.07 g Tecophilic SP-80A-150 dissolved in 38 mL CHCl₃ on rollermill, 19.975 g soln removed (1.0 g Tecophilic) and mixed with 0.0589 g IRP64-Ag by hand. Poured on release liner to allow CHCl₃ to evaporate.
*29. Tecoflex EG-80A polyurethane with IRP64-Ag*
   5.012 g Tecoflex MG-8020 dissolved in 64.209 mL CHCl₃ on rollermill, 20.08 g soln removed (1.0 g Tecoflex) and mixed with 0.0528 g IRP64-Ag by hand. Poured on release liner to allow CHCl₃ to evaporate.
*30. Preparation of MAC-3-Ag*
   10.29 g of Dowex MAC-3 (The Dow Chemical Company, Liquid Separations, Midland, MI 48641-1206) material was slurried into 150CC of 0.1N NaOH solution for 20 minutes and the MAC-3 filtered rinsed until the filtrate was neutral pH and the solid slurried in 150CC deionized water and 8.5g of silver nitrate (Fluka) added and the mixture stirred for 1.5 hours in the absence of light. The material was filtered, washed and dried under vacuum at 130°C.
*31. MED-4955 liquid silicone rubber with MAC-3-Ag*
   8 g part A + 8 g part B + 0.5105 g MAC-3-Ag (3 % w/w) mixed in Speedmixer, spread on release liner and placed in vacuum oven at room temperature to remove air bubbles. Another release liner placed on top, rolled with a jar to flatten and then cured (∼80 °C, ∼10 min). Cure was complete.
31. *Preparation of MAC-3-Cu and Amberlite IRP64-Cu*
   Dowex MAC-3 (The Dow Chemical Company, Liquid Separations, Midland, MI 48641-1206) or Amberlite IRP64 (Rohm and Haas Company, a subsidiary of Dow Chemical Company, Philadelphia, PA 19106-2399) weak cation exchange material (Na⁺ form) was stirred in a minimal amount of deionized water and a large excess (∼10-500molar excess) of copper sulfate (Cu(SO₄)₂) and the mixture stirred for 1 hour at room temperature using a mechanical stirrer. The (blue-colored) salt was filtered and rinsed with deionized water until the filtrate was clear (no blue). The salt was dried at 130°C under vacuum and used in the formulation of silicone rubber materials. Neither MAC-3-Cu nor Amberlite IRP64-Cu inhibited the cure of silicone elastomers (Momentive Performance Materials UV-curing silicone (2060) or Nusil MED-4955.
*32. Preparation of salts of crosslinked polycarboxylated weak cation exchange materials (General Procedure).*
   Amberlite IRP64 and MAC-3 weak cation exchange material was stirred in a minimal amount of deionized water and a large excess (∼10-500molar excess) of the salt (containing the cation of interest, such as benzalkonium chloride) was added and the mixture stirred by the addition of a mechanical stirrer for 60 minutes. The solid was filtered washed with copious amounts of deionized water (until the filtrate does not contain any benzalkonium chloride (for example) as evidence from ultraviolet spectroscopic evaluation of the filtrate. The modified IRP64/MAC-3 was dried under vacuum at 130°C and the material was ground with the aid of an IKA homogenizer and the powder put through a sieve with a 35 micron cutoff. The dried powder was dried under vacuum and used for addition to various polymer formulations.
33. Preparation of Amberlyst A21-Acetylsalicylic acid (ASA) Amberlyst A21 and acetyl salicylic acid (ASA) were stirred together at room temperature in a solution of water/isopropyl alcohol (2:1) for 12 hours and the product filtered, washed with water and soxhlet extracted with isopropyl alcohol (12 hours). The product (Amberlyst A21-ASA) was air and vacuum dried, milled to 5 micron particle size and incorporated into LSR silicone rubbers Performance Materials UV-curing silicone (2060) and Nusil MED-4955. Both materials cured as expected and the release of ASA from the silicone materials was monitored by UV spectroscopy with exposure of the material to PBS solution.
The above samples, with the exception of the Amberlyst A21-ASA, were used to evaluate antimicrobial effectiveness.

In compliance with the statute, embodiments of the disclosure have been described in language more or less specific as to structural and methodical features. It is to be understood, however, that the entire invention is not limited to the specific features and/or embodiments shown and/or described, since the disclosed embodiments include forms of putting the invention into effect. Tables 1 and 2 exemplifies some of the compositions prepared is shown below.

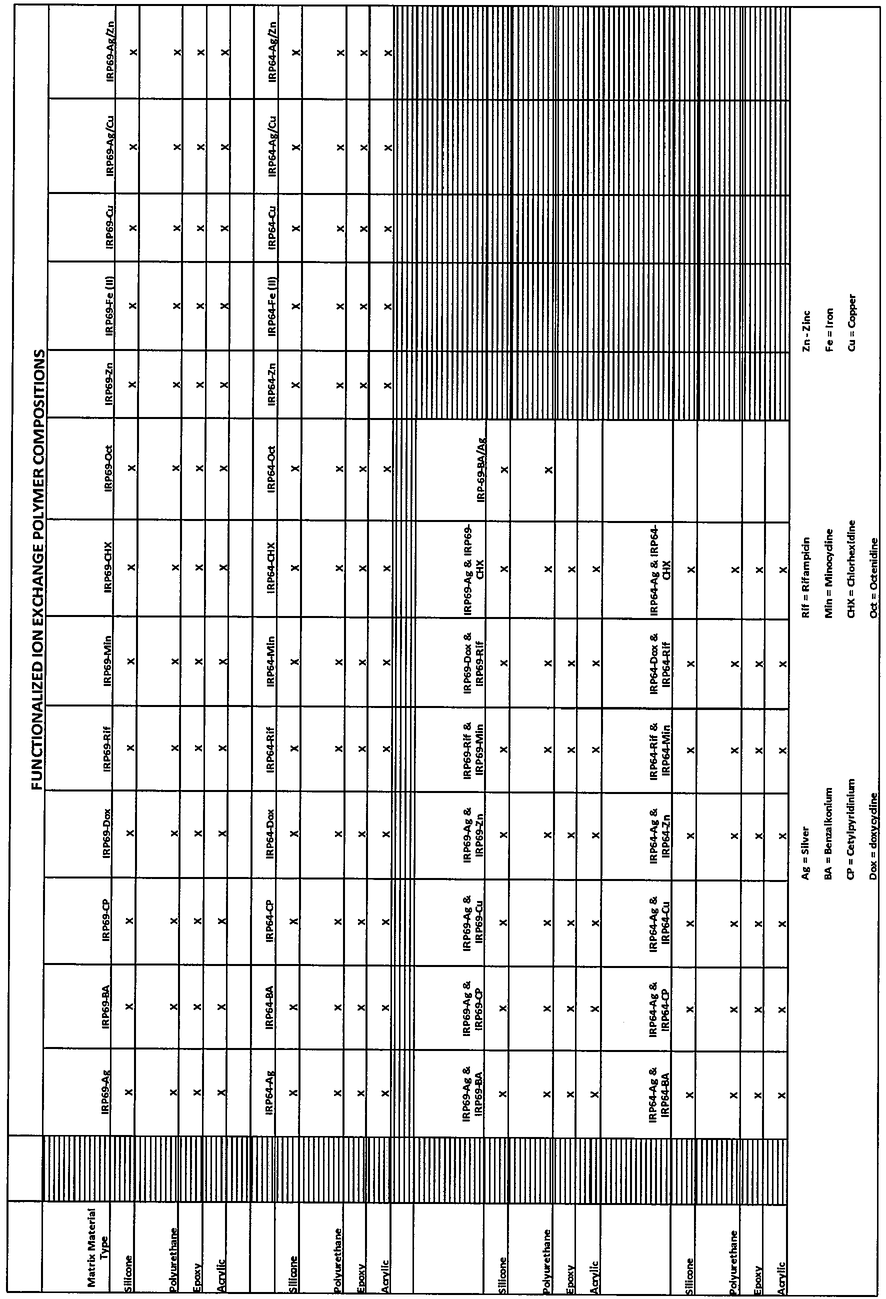

## Claims

1. A composition comprising:
a polymer matrix; and
a plurality of ion-exchange particles distributed within the matrix.

2. The composition of claim 1 wherein the polymer matrix comprises one or both of a thermoplastic or thermoset polymer.

3. The composition of claim 1 wherein the polymer matrix comprises one or more of a polyalkylene, polysiloxane, polyamide, epoxy, polycarbonate, polyester, polyol, polyarylene, vinyl polymer, acrylic polymer (polyacrylonitrile, polyacrylate), asphalt, bitumen, polysaccharide, cellulosic, and/or polyurethane.

4. The composition of claim 1 wherein at least some of the plurality of ion-exchange particles comprises one or more of styrene, acrylic, vinyl, sulfonate, quaternary ammonium, amine, and/or carboxylate.

5. The composition of claim 1 further comprising a counter-ion material chemically associated with at least some of the ion-exchange particles.

6. The composition of claim 5 wherein the biologically active counter-ion material is therapeutically efficacious.

7. The composition of claim 5 wherein the counter-ion material comprises one or both of inorganic and organic cations and/or anions.

8. The composition of claim 5 wherein the biologically active counter-ion material comprises one or more of mono, di, and/or trivalent cations.

9. The composition of claim 5 wherein the biologically active counter-ion material comprises Ag+, Cu++, Ba++, Zn++, Ga+++, Fe++, and Ca++, Mg++, and Ce+++ .

10. The composition of claim 5 wherein the counter-ion material comprises one or more of a metal cation, quaternary ammonium, organics ions, protonated amines, carboxylate, phosphate, and/or biguanides.

11. A solid polymeric composition production method comprising:
providing a plurality of ion-exchange particles;
providing polymer precursors in a substantially unsolidified state;
prior to solidifying the precursors, blending the precursors with the ion-exchange particles to form a mixture; and
solidifying the mixture to form a solid polymeric composition.

12. The polymeric material production method of claim 11 wherein the providing the plurality of ion-exchange particles comprises milling a hardened polymer and processing the hardened polymer to provide a plurality of particles ranging in size from about 0.05 microns to about 2500 microns.

13. The polymeric material production method of claim 12 wherein the hardened polymer comprises styrene, acrylic, sulfonate, carboxylate, amine, and/or quaternary amine functionalities.

14. The polymeric material production method of claim 11 wherein the providing the polymer precursors in a substantially unsolidified state comprises providing nonvulcanized silicone rubber precursors.

15. The polymeric material production method of claim 11 wherein the blending the precursors with the ion-exchange particles to form a mixture comprises providing the particles in an amount between from about 1 to 75% weight percent of the mixture.

16. The polymeric material production method of claim 11 wherein the solidifying of the mixture to form a solid polymeric composition comprises evaporating solvent from a polymer lacquer to form the solid polymeric composition.
